# EUROPEAN PATENT SPECIFICATION

(11) **EP 2 673 001 B1**
(45) Date of publication and mention of the grant of the patent: **21.12.2016**
(21) Application number: 12737597.0
(22) Date of filing: 10.02.2012
(51) Int. Cl.: A61K 48/00, C07K 7/08, C12N 9/12, A61K 47/48

(54) **NTS-POLYPLEX NANOPARTICLES SYSTEM FOR GENE THERAPY OF CANCER**
NTS-POLYPLEX NANOPARTIKELSYSTEM ZUR GENTHERAPIE VON KREBSERKRANKUNGEN
SYSTÈME DE NANOPARTICULES NTS-POLYPLEX S'APPLIQUANT À LA THÉRAPIE GÉNIQUE DU CANCER

(30) Priority: 10.02.2011 MX 2011001558
(43) Date of publication of application: 18.12.2013
(73) Proprietor: Centro de Investigacion y de Estudios Avanzados del Instituto Politécnico Nacional, México, D.F. 07360 (MX)
(72) Inventor: CASTILLO RODRÍGUEZ, Rosa Angélica, Aguascalientes Aguascalientes 20190 (MX); ESCOBEDO SÁNCHEZ, María de Lourdes, Tlalnepantla Estado de México 54198 (MX); MARTÍNEZ FONG, Daniel, México D.F. 07300 (MX)
(74) Representative: Germinario, Claudio
(86) International application number: PCT/IB2012/050619
(87) International publication number: WO 2012/107908

(56) References cited:
- WO-A2-2007/093373
- MX-A- PA01 001 256
- H A RUBIO-ZAPATA ET AL: "NT-polyplex: a new tool for therapeutic gene delivery to neuroblastoma tumors", CANCER GENE THERAPY, vol. 16, no. 7, 1 July 2009 (2009-07-01), pages 573-584, XP55041633, ISSN: 0929-1903, DOI: 10.1038/cgt.2009.1
- ARANGO-RODRIGUEZ M L ET AL: "Biophysical characteristics of neurotensin polyplex for in vitro and in vivo gene transfection", BIOCHIMICA ET BIOPHYSICA ACTA - GENERAL SUBJECTS, ELSEVIER SCIENCE PUBLISHERS, NL, vol. 1760, no. 7, 1 July 2006 (2006-07-01) , pages 1009-1020, XP025014910, ISSN: 0304-4165, DOI: 10.1016/J.BBAGEN.2006.02.021 [retrieved on 2006-07-01]
- SOUAZÉ FRÉDÉRIQUE ET AL: "Expression of neurotensin and NT1 receptor in human breast cancer: a potential role in tumor progression", CANCER RESEARCH, AMERICAN ASSOCIATION FOR CANCER RESEARCH, US, vol. 66, no. 12, 15 June 2006 (2006-06-15) , pages 6243-6249, XP002518143, ISSN: 0008-5472, DOI: 10.1158/0008-5472.CAN-06-0450
- VIVES E ET AL: "Cell-penetrating and cell-targeting peptides in drug delivery", BBA - REVIEWS ON CANCER, ELSEVIER SCIENCE BV, AMSTERDAM, NL, vol. 1786, no. 2, 1 December 2008 (2008-12-01), pages 126-138, XP025742020, ISSN: 0304-419X, DOI: 10.1016/J.BBCAN.2008.03.001 [retrieved on 2008-04-09]
- SOMAÏ SONIA ET AL: "Neurotensin counteracts apoptosis in breast cancer cells", BIOCHEMICAL AND BIOPHYSICAL RESEARCH COMMUNICATIONS, ACADEMIC PRESS INC. ORLANDO, FL, US, vol. 295, no. 2, 12 July 2002 (2002-07-12) , pages 482-488, XP002518144, ISSN: 0006-291X, DOI: 10.1016/S0006-291X(02)00703-9

## Description

### Field of the invention.

The invention relates to the field of antineoplastic gene therapy, which comprises the specific introduction of a functional gene into a cancer cell type. More specifically, the present invention relates to strategies for delivering this transfer of genetic material *in vivo* by using nanoparticles that carry one gene whose expression is responsive to therapeutic purposes, which are internalized, for example, in breast cancer cells to prevent the progression and metastasis of the disease.

### Background of the invention.

Breast cancer is the leading cause of death among females worldwide. It is estimated that of the women who live to be 85 years old, one in nine will get the disease some time in her life. Breast cancer appears at higher rates in developed countries, although breast cancer incidence rates have been rising in developing countries shortening the difference. According to data from the World Health Organization (WHO), the United States, France, Iceland, Great Britain, Canada, the rest of Europe and Australia show the highest incidence rates of invasive breast cancer; while in underdeveloped countries the rates may be underestimated. In U.S., it is estimated that one in eight women will develop invasive breast cancer sometime in her life. The incidence of the disease has increased dramatically; for example, it is estimated that in 2010 about 40,000 women will die from this cause in the U.S. only. The cancer risk is doubled if a female has a family history. Five to ten percent of breast cancers are linked to BCRA1 and BCRA2 genes, since women with these genes have an 80% risk of suffering the disease. However, 70 to 80% of breast cancers occur without a family history, which means that the causes are environmental aside from age (http://www.who.int).

The variety of treatments for breast cancer has increased and includes: the use of aromatase inhibitors, which block an enzyme involved in the synthesis of estrogen and progesterone; the use of monoclonal antibodies, which prevent the activation of cellular receptors as HER2 and IGF-1; the use of kinase inhibitors, which inhibit cell signaling through the receptors; vaccines, which stimulate the production of specific antibodies for tumor proteins and can be of cells or peptides; and other treatments that include inhibitors of different proteins as well as gene therapy that alters the manufacturing of cellular proteins. Gene therapy emphasizes the use of E1A (the expression of adenoviral E1A region functions as an inhibitor of tumor genes, resulting in a cell differentiation and induction of apoptosis of cancer cells, besides sensitizing cancer cells to chemotherapeutic agents; oncolytic adenovirus are used) and Ad5CMV-p53, which is a failed replication adenoviral vector encoding the native gene p53, so that it induces apoptosis. Malignant mesothelioma is a rare form of cancer that starts in the mesothelium, the membrane that covers and protects most internal organs. The mesothelium is formed by two layers, one that surrounds the organ itself and another that form an epithelial sac around it. Pleural mesothelioma develops in the lining of the lungs called pleural membrane and is composed of mesothelial cells in two layers: the parietal and the visceral layer. Normally, a small amount of fluid between these two levels is produced, which lubricates the movement of the organs protected. When normal cells of the mesothelium lose control and divide quickly, a mesothelioma appears. The most common form of mesothelioma is "pleural" mesothelioma; other forms are "peritoneal" mesothelioma, which affects the lining of the abdominal cavity, and "pericardial" mesothelioma affecting the lining of the heart. The incidence of mesothelioma in Western Europe is expected to reach its peak between 2010 and 2020. Those who have worked directly with asbestos or asbestos products have a higher risk of developing mesothelioma; however, there have been cases of mesothelioma in people with minimal exposure to this product (Pass, I. *et. al*., 2005). Any form of mesothelioma is very aggressive and often resistant to treatment. In addition, early diagnosis is rare, so the treatment of mesothelioma usually fails in offering a complete cure. But with the development of new drugs and early detection techniques, the outlook is improving with the hope to effect at least patient's survival. Patients with pleural mesothelioma have three options: surgery, chemotherapy, and radiation therapy. Normally, patients receive a combination of two or more of these types of treatment (Pass, I. *et. al*., 2005).

Early detection of pleural mesothelioma and breast cancer improves patient's prognosis greatly, and these patients have a wider range of treatment options. If the disease is diagnosed timely, the surgery to remove tumors followed by chemotherapy or radiation therapy to eliminate remaining cancer cells can be a successful treatment. Patients who are not diagnosed early have fewer options, and these are mostly limited to a palliative care to relieve the pain and other symptoms of the disease, and improving the quality of life. Therefore, it is necessary to have more efficient alternatives for treating cancer of any stage, including refractory and metastatic stage.

Gene therapy is a new medical strategy for treating various serious disorders such as tumor diseases. In gene therapy viral vectors and nonviral and bacterial methods (Patyar S., *et. al*., 2010) have been developed for transferring genetic material *in vivo* and *ex vivo.* Viral and bacterial methods are efficient, but represent a risk to patients. By contrast, non viral methods such as synthetic polymers, liposomes, and electroporation generally have lower transfection efficiency, but are a safer therapy.

The present invention relates to a safer and more efficient nonviral strategy for genic therapy. The strategy is based on the natural cell process of receptor-mediated endocytosis by which the transfer of genetic material is specifically targeted to the cell of interest (target cell) where the binding of a ligand to its receptor occurs on the cell surface producing endocytosis. Here, the ligand is chemically modified, forming a complex carrying the DNA linked covalently to a polycation (as polysinas or protamines); alternatively, a fusogenic peptide (as hemagglutinin HA2) and a charyophillic peptide (as Vp1 of SV40) can be coupled thereto. Once endocytosed, the complex reaches the endosomal compartment, and thus the genetic material introduces itself into the nucleus. More specifically, the present invention relates to a strategy of gene therapy to eliminate cells involved in breast cancer and pleural mesothelioma, and involves a targeted delivery of genes using the molecular complex that is internalized directly and exclusively in cells affected (or target cells). This molecular complex functions as a gene carrier and is a biological complex called neurotensin polyplex or NTS-polyplex, which is a biodegradable nanoparticle system that transfers therapeutic genes or suicide genes to cell populations expressing receptor 1 with high affinity to NTS (NTSR1 or NTS1, which has been described by Martinez-Fong et.al. (Patent MX264932B, 2001; Molecular Brain Research, 2002; Biochemica et Biophysica Act, 2006).

In the state of the art, the technologies used for targeted gene delivery are based on the natural process for receptor-mediated encocytosis in the cell surface by *in vitro* and *in vivo* essays as those mentioned below:
Patent EP0587738B1 describes a soluble molecular complex for the genes shipment encoding secretory proteins in directed way. These molecular complexes are defined by their ability to release the gene in intracellular conditions and its components are: a gene-binding agent constituted by a polymer that condenses the DNA, and a cell-specific binding agent. It exemplifies the polycation including polyysine as the gene-binding agent, and mentions that the cell-binding agent can be a surface receptor, illustrating asialoglycoprotein to direct genes to hepatocytes via the asialoglycoprotein receptors. It also delineates an albumin gene transfection into hepatocytes in a pre clinical model of analbuminemic rat. The system described in this patent shows low transfection efficiency because the endosomes for asialoglycoproteins are destined to bind with a lysosome to degrade their contents.
Patent US5830852 describes a gene delivery system of DNA to cells that specifically bear the insulin receptor which includes a nucleic acid binding peptide, H₂N-Thr-Lys₁₈-(S-Acetimidomethyl-Cys)-COOH linked to insulin or insulin derivative, and associated with condensed nucleic acid encoding for sequences of therapeutic benefit. *In vitro* essays are exemplified with a cellular line of hepatocytes. However, there are evidences that the levels of transfection with this receptor fail to outperform conventional transfection methods. The strategy described seems therefore, not suitable for pharmaceutical application.
Chen J. and col. (1994) describes a gene delivery system using endocytosis mediated by epidermal growth factor (EGF) receptor. The system comprises a conjugate that includes poly-1-lysine attached to a monoclonal antibody, which specifically binds to the EGF receptor to be transfected into the cell. The conjugate is akin to the DNA plasmid that possesses the gene of interest. Tests showed are cell assays *in vitro* and the expression demonstration of a reporter gene.
Lactosilated poly-1-lysine and galactosilated hystones have also been used for the intracellular delivery of DNA (Midoux, P. *et. al*., 1993; Chen J. *et. al*., 1994).

However, for the molecular complexes above described to be efficiently released into the cytoplasm a pharmacological agent that breaks the endosome membranes such as chloroquine must be added to the system. Such systems are therefore, not suitable for use *in vivo.* NTS-polyplex complex has also been used to transfect genes *in vivo,* which is close to its application in gene therapy. Rubio-Zapata and col. (2009), describes the subcutaneous transplantation of N1E-115 cells (2.5 X 10⁶ cells/200 µL) in Nu/Nu mice, generating a model of neuroblastoma, determining the time course of tumor growth, the macroscopic parameters and histopathologic features. Confocal microscopy analysis showed *in vivo, in situ*, and *in vivo* the internalization of NTS-polyplex marked with propidium iodine in the cell line GFP-NTSR1-N1E-115 expressing the NTSR1 receptor fused with the green fluorescent protein. It also shows that NTS-polyplex can transfect HSV-Tk suicide gene into allogeneic neuroblastoma transplants and creates the desired antitumor therapeutic effect. Consequently, additional administration of ganciclovir (GCV) to Nu/Nu mice (75 mg/kg body weight/day) significantly reduces tumor progression in mice transfected locally or intravenously, compared with the control group transfected with empty plasmid, i.e., lacking the HSV-TK gene (Rubio-Zapata, H.A. *et al*., 2009).

Also, Martinez-Fong and col. (2006) described that in the presence of the receptor NTSR1 in dopaminergic neurons of the *substantia nigra* it is possible to use NTS-polyplex complex for treating the neuronal degeneration in Parkinson's disease.

These data strongly support the specificity of transfection of NTS-polyplex when innoculated *in situ*, so that after determining its bioavailability and biosecurity NTS-polyplex can be used in gene therapy protocols in mammals, including humans.

The present invention emphasizes the transfer of suicide genes specifically through the receptor NTSR1 endocytosis. Classical chemotherapy has been established as a standard treatment for patients with cancer by delaying the development of several types of tumors. However, chemotherapy treatments do not specifically target malignant cells and its toxicity also affects healthy cells. By contrast, the use of the NTS-polyplex nanoparticles system in molecular chemotherapy protocols, such as that described in the present invention, offers superior advantages by its high specificity on tumor cells expressing the receptor NSTR1.

### Embodiments of the invention.

The present invention is defined in the claims.

The present invention is based on the expression of the high-affinity neurotensin (NTS) receptor (NTSR1), which is induced in breast invasive ductal adenocarcinoma, a cancer with the highest incidence and mortality rate worldwide. The present invention relates to the development of a new antitumor therapy for cells expressing NTSR1 using the gene-transference system known as NTS-polyplex.

One objective of the invention relates to determining the ability *in vitro* of MCF7 and MDA-MB-231 cell lines, both of breast ductal adenocarcinoma, to be transfected by NTS-polyplex as a first approximation to antitumor therapy, and the effectiveness of the invention system to transfect xenoimplanted MDA-MB-231 to nude immunodeficient mice with a subsequent significant decrease in tumor development.

The main technological contribution of the present invention relates to the strategy for transferring genes into breast tumoral cancer cells to destroy them, based on the nanomolecular complex called NTS-polyplex specially designed for treating breast cancer or pleural mesothelioma. The NTS-polyplex is characterized because it possesses a plasmid (pDNA) electrostatically linked to a chariophillic peptide (PK), also electrostatically linked to poly-L-lysine (PLL) conjugated to the neurotensine (NTS) and to a fusogenic peptide (PF), where the pDNA has the coding sequences of a therapeutic gene, wherein said fusogenic peptide chemically coupled to poly-L-lysine consists of the following sequence GLFEAIAEFIEGGWEGLIEGSAKKK.

A preferred embodiment of the invention relates to the above described NTS-polyplex complex that possesses a specific gene for treating breast cancer.

Another embodiment of the invention relates to using NTS-polyplex complex that transfers here a pDNA encoding a suicide gene, whose expression produces the destruction of cancer cells and therefore works for treating breast cancer, including invasive or highly invasive breast cancer, where the treatment comprises the systemic administration of the complex, parenterally or intravenously, and where after transfecting the malignant or transformed cells that form the cancerogenous tissue of the breast or target cells, occurs the systemic destruction of them, and the pDNA can also carry transcription promoters of specific tissues.

Another embodiment of the present invention relates to using NTS-polyplex complex in molecular chemotherapy trials for breast cancers, consisting of the transfection of a suicide gene for a targeted therapy by transfecting an enzyme that acts on a pro-inactive drug (activated by effect of said enzyme); the suicide gene can be the mentioned HSV-Tk. However, there are many prodrugs that can be converted by effect of the enzyme encoded (suicide gene), and the system can be selected depending on the type of tumor. When using HSV-Tk, the treatment also comprises the administration of Ganciclovir (GCV) at 24 hours post-transfection and a continued, therapeutically effective dosage-regimen. Transfection of a toxic gene is itself an embodiment of the invention. In another embodiment of the invention, NTS-polyplex can carry a gene that is not toxic by itself, but is toxic for the transformed cell, so the specificity of the strategy becomes even thinner and is restricted to tumor cells.

Salmons, B. and col. (2010) reviewed the twenty-eight suicide gene-prodrug systems that have been described to date. Some of these suicide genes and prodrugs, such as HSV-Tk/Ganciclovir, Cytosine deaminase/5-fluorcytosine, Cytochrome P450/cyclophosphamide or ifosfamide, have been use in phase I clinical trials, using viral vectors, or encapsulated cells. The NTS-polyplex, as does Tk/Ganciclovir suicide gene therapy, represents a feasible alternative for the delivery of the mentioned suicide genes and prodrugs.

A particular objective of the invention is the use of NTS-polyplex complex to prevent the development of invasive cancer, where the pDNA delivers and transfects any of the following genetic materials: a tumor suppressor gene (TSG) that restores the gene that is lost; an anti-oncogenic gene; nucleotide sequences to disrupt the expression of the amplified oncogenes; a proapoptotic gene or a immunomodulation gene; and it can also deliver tissue-specific transcription promoters.

Also, a further embodiment of the invention is the use of NTS-polyplex where the therapeutic gene, including the suicide gene, can be regulated by a hybrid promoter composed of alpha-1 elongation factor promoter and 5' untranslated region of leukemia virus of human cells. Another embodiment of the invention relates to the possibility of using the complex to treat breast cancer, where the treatment comprises the administration *in situ* of such complex. Another embodiment of the gene-therapy strategy of the invention relates to a combination of the treatment of the invention with conventional chemotherapy or radiotherapy, which can potentiate or reaffirm the desired result.

It is a further objective of the present invention to provide a NTS-polyplex for treating breast malignant cancer by gene therapy, wherein this cancer is characterized for being insensible to conventional treatments.

The NTS-polyplex complex of the invention can also be used to induce specific apoptosis; for example, through p53, the TNF inducing ligand related with apoptosis (TRAIL/Apo2L), the co-expression of Bax/Bc12 or Bac/Bc12, etc. The NTS polyplex of the invention can also be used to promote the expression of superantigens that induce an effective immune response, or the application of cutting-edge technology such as Spliceosome-mediated pre-RNA *trans*-splicing

### (SMART™.)

A further objective of the invention relates to the possibility of using the NTS-polyplex to evaluate the physiological effects of transgene expression in breast cancer, including studies of pharmacokinetics, bioavailability and biosafety, also including the possibility of using a cancerous cell line of mammary origin such as the cell line MDA-MB231, which can be transplanted to nude mice (Nu/Nu) as an experimental model.

### Brief description of the figures.

- **Figure 1.**: Shows micrographs of electronic microscopy of transmission showing DNA plasmid in its relaxed form (A) and compacted into nanoparticles with a diameter of 100 nm, known as NTS-polyplex (B).
- **Figure 2.**: Shows the plasmid pEGFP-N1 (4.7 Kpb) encoding green fluorescent protein (GFP) under the control of the cytomegalovirus (CMV) promoter (Clontech, Palo Alto, CA, USA.)
- **Figure 3.**: Shows the plasmid pTracer-EF/V5-His (5.93 Kpb) encoding green fluorescent protein (GFP) under the control of cytomegalovirus (CMV) promoter. It also has the EF-α promoter, which provides a high-level expression in mammal cell lines and controls the expression of a gene of interest (Invitrogen, Carlsbad, CA, USA.)
- **Figure 4.**: Shows the plasmid pORF-LUc (4.896 Kpb) encoding luciferase enzyme (Luc), obtained by cloning Luc cDNA (1.656 Kpb) in the sense orientation Ncol-Nhel of the expression vector pORF. The Luc cDNA was earlier obtained from pGL3-Basic vector (Promega Corporation, Madison, WI) using Nco1 and Xba1 enzymes.
- **Figure 5.**: Shows the plasmid pORF-HSVTK (4.373 Kpb.) encoding thymidine kinase (TK) enzyme of HSV and is under a hybrid promoter constituted by the elongation factor-1α promoter and the 5' untranslated region of the human T-cell leukemia virus (InvivoGen, San Diego, CA, USA.)
- **Figure 6.**: Shows the RT-PCR analysis showing the presence of mRNA for NTSR1 in MDA-MB-231 and MCF7 cell lines. The NIE-115 cells are the positive control, and the L929 cells are the negative control.
- **Figure 7.**: Shows an immunofluorescence against NTSR1 showing its presence in the cell lines MDA-MB-231 and MCF7 counterstained with Hoechst. Calibration bar = 25 µm.
- **Figure 8.**: Shows the functionality of NSTR1 for specific gene transfer by NTS-polyplex in MDA-MB-231 cells. Note the internalization of NTS-polyplex (see A to D), which was blocked with 1 µM Neurotensin (NTS) (see E to H), with 500 nM of SR48692 (see I to L), or 0.45 M sucrose (see M to P). Calibration bar = 20 µm.
- **Figure 9.**: Shows the functionality of NTSR1 for specific gene transfer by NTS-polyplex in MCF7 cells. Note the internalization of NTS-polyplex (see A to D), which was blocked with 1 µM Neurotensin (NTS) (see E to H), 500 nM of SR48692 (see I to L), or 0.45 M sucrose (see M to P). Calibration bar = 20 µm.
- **Figure 10.**: Shows the expression of green fluorescent protein (EGFP-N1) in the cell lines MDA-MB-231 and MCF7 transfected with pEGFP-N1 plasmid using NTS-polyplex. Calibration bar = 20 µm.
- **Figure 11.**: Shows the detection of early stage apoptosis in the cell line MDA-MB-231 transfected with pORF-HSVTK plasmid using NTS-polyplex.
- **Figure 12.**: Shows the effect of transfection of pORF-HSVTK plasmid on the feasibility of different cell lines using NT-polyplex. CP = primary culture of human breast healthy tissue. GCV = ganciclovir adjunct treatment. The mean ± S.E.M. was obtained from three independent experiments. * Statistically significant difference when compared with control. P < 0.005, Dunnett test.
- **Figure 13.**: Shows the toxicity inhibition of the transfection of NTS-polyplex in MDA-MB-231 cells by the activation blockers of NTSR1. The GCV (10 µg/mL) was present from the third post-transfection hour. Control = transfected cells with NTS-polyplex pORF-HSVTK without blocking. The media ± S.E.M. was obtained from three independent experiments. *Statistically significant difference when compared with control. P<0.005, Dunnett test.
- **Figure 14.**: Shows the animal model of breast cancer. The tumor (indicated by the arrow) was generated by xenotransplantation of 3 million MDA-MB-231 cells in the subcutaneous layer of the right flank of athymic 4-wk old female Nu/Nu mice.
- **Figure 15.**: Shows the growth curve of tumors generated by the xenograft of MDA-MB-231 cells in the subcutaneous layer of the right flank of athymic 4-wk old female Nu/Nu mice.
- **Figure 16.**: Confocal microphotographs showing the internalization of NTS-polyplex marked with propidium iodide in MCA-MB-231 cells expressing the NTSR1-GFP. The NTS-polyplex was transfected intratumorally.
- **Figure 17.**: Confocal microphotographs showing the expression of green fluorescent protein (GFP) in MDA-MB-231 cells. The NTS-polyplex was transfected intratumorally.
- **Figure 18.**: Confocal microphotographs showing the expression of green fluorescent protein (GFP) in MDA-MB-231 cells. The NTS-polyplex was blood transfected.

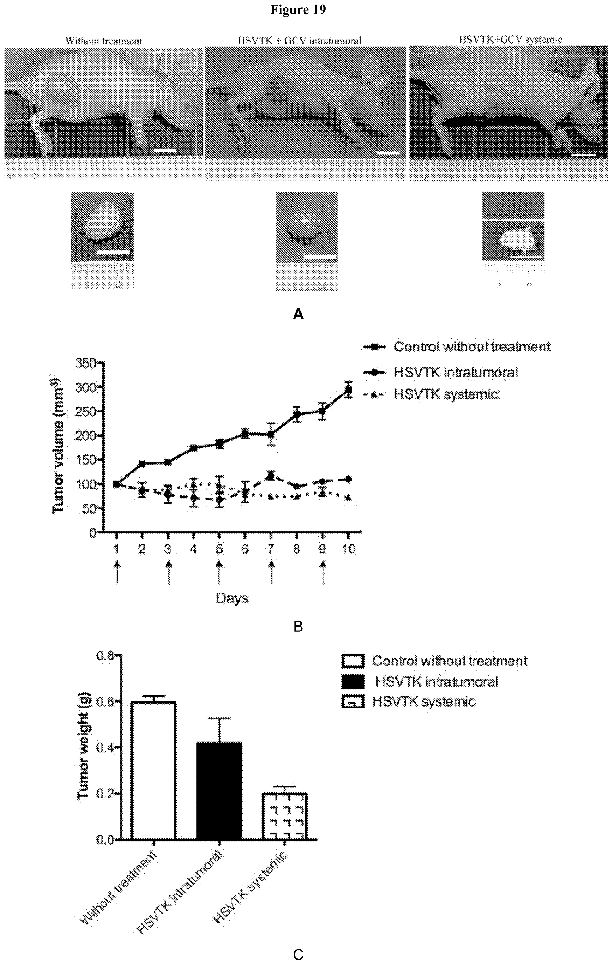

- **Figure 19.**: Shows the decrease of tumor growth by NTS-polyplex transfection of a HSVTK suicide gene and ganciclovir (GCV) treatment. The transfections were done every third day (arrows) and GCV (100 mg/kg of corporal weight, i. p.) was administered daily. Representative photographs of animals without treatment (control) or treated with intratumor transfection and systemic transfection (A; upper panel) and photographs showing the dissected tumors from those animals (A, inferior panel). Graphs of tumor-growing rate (B) and tumor weight (C) at the end of the study. Calibration bar = 1 cm. n = 3 independent experiments.

### Detailed description of the invention.

The present invention is a technological contribution that refers to the possibility of genetically modifying breast carcinoma cells using a molecular complex specially designed to direct the suitable genes to altered target cells. This possibility stems from the approach and development of a strategy that combines the design of gene vectors or carriers with the properties found in the target cells. The present invention relates to the demonstration of the directed gene delivery through internalization of the receptor NTSR1. The internalization of the receptor NTSR1 present in specific cells is activated by NTS, being most useful as a transference route of reporter and therapeutic genes to cell populations expressing this receptor *in vivo,* and which are affected by the disease.

The present invention raises the possibility of using the complex NTS-polyplex; for example, for gene therapy protocols to treat breast cancer or pleural mesothelioma. The strategy is a sum of elements that enhance the performance expected of NTS-polyplex. The therapeutic strategy here proposed is based on the presence of receptors NTSR1 in two cell lines whose origin is breast cancer, since the receptor NTSR1 undergoes endocytosis. The strategy has a better chance of success than conventional therapies. However, it is possible to use both therapies. In this sense, the oncologist will be able to develop a treatment schedule indicating how to diversify the treatments depending on the conditions and needs of the patient. Also, the doses and preferred routes of administration can be set by the specialist. The advantage of the present invention is that irrespective of the route of administration, the included therapeutic genes are targeted to cancer cells, especially those characterized by being highly invasive without affecting healthy cells. The conclusive evidence on this high specificity is described below.

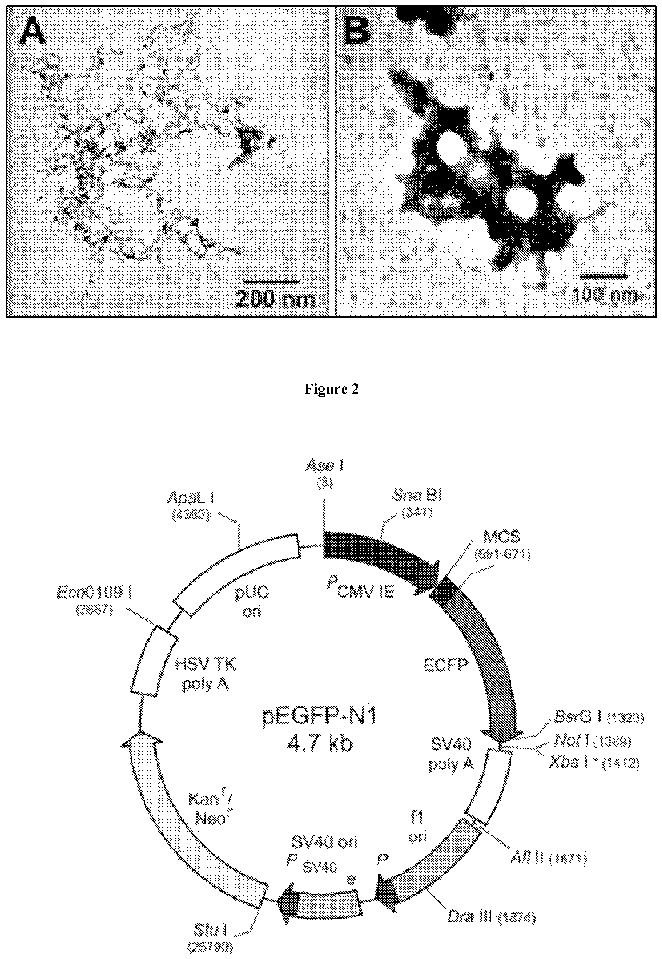

The gene vector or carrier used in the present invention is called NTS-polyplex, which is a complex comprised by biodegradable molecules, toroidally shaped, with an average diameter of 100nm, and are programmed to transfer genes (transfection) for experimental or therapeutical utility in cells expressing high affinity receptor for neurotensin (NTSR1) (Fig. 1.) These nanoparticles are formed by attaching electrostatically a plasmid (pDNA) with a charyophillic peptide (PK) and poly-L-lysine (PLL) conjugated to the neurotensine (NTS) and to a fusogenic peptide (PF) wherein said fusogenic peptide chemically coupled to poly-L-lysine consists of the following sequence GLFEAIAEFIEGGWEGLIEGSAKKK. The conjugate is known as the NTS carrier. The electrostatic binding occurs, because the pDNA is a polyanion, and both the PK, and the PLL are polycations. The Martinez-Fong research group recently increased the efficiency of NTS-polyplex by coupling the fusogenic domain of the HA2 peptide of hemagglutinin (PF) and the charyophillic domain PK of Vp1 SV40 peptide; the cellular functions of the PF and PK would the rescue of NTS-polyplex from the acidic endosomes and routing the plasmid to the cell nucleus, which may explain the high efficiency of the transfeccion (Martinez-Fong, *et. al*., 2006).

The NTS in the carrier is the molecule responsible for activating endocytosis in the receptor NTSR1, while the poly-L-lysine (polycation) will electrostatically bind the plasmid (polyanion) and compact it in toroidal nanoparticles to be endocytosed without affecting its specificity.

The inclusion of eukaryotic constitutive promoters such as α1 elongation factor promoter, or tissue-specific promoters such as dopamine transporter (DAT) promoter in NTS-polyplex has resulted in the prolonged expression of the transfected gene *in vivo* (Martinez-Fong, D. *et. al*., 2002; 2006).

Each of the components of NTS-polyplex performs a particular function in the gene-transfer process. PLL is responsible for condensing pDNA into 100 nm nanoparticles, while NTS reaches NTSR1 receptor and joins it to induce endocytosis of NTS-polyplex. PF, when activated by the acidity of the endosome, supports the transfer of NTS-polyplex to the cytoplasm by rescuing it from lysosomal degradation. Once in the cellular compartment, PK acts transporting pDNA into the nucleus to transcribe the coding sequence of the gene of interest, and subsequently translate it into a functional protein in the ribosomes. To further increase the specificity of transfection the pDNA is added with a tissue-specific promoter, which is responsible of directing the expression of the transgene only to those cells with the transcriptional elements that activate the promoter. Taken together, all these cell instructions are programmed in the NTS-polyplex components, and only the transfection target cell is able to decipher them to express the experimental or therapeutical protein of interest. A minor error in the chemical composition of only one component of the NTS-polyplex makes inoperative the NTS-polyplex programming.

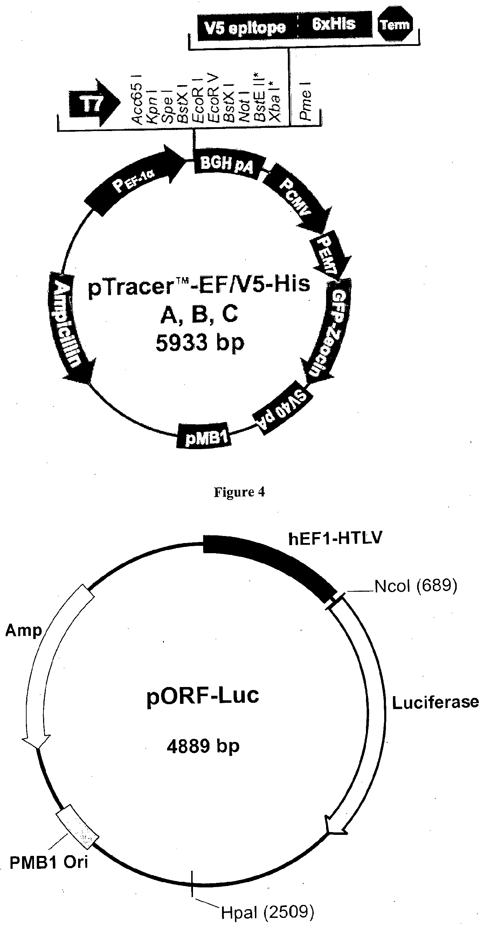

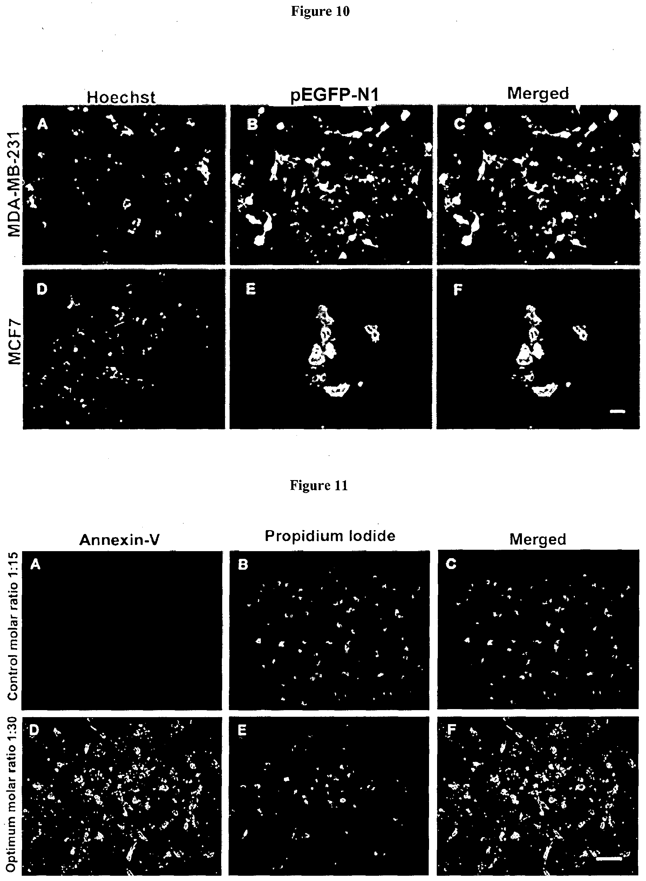

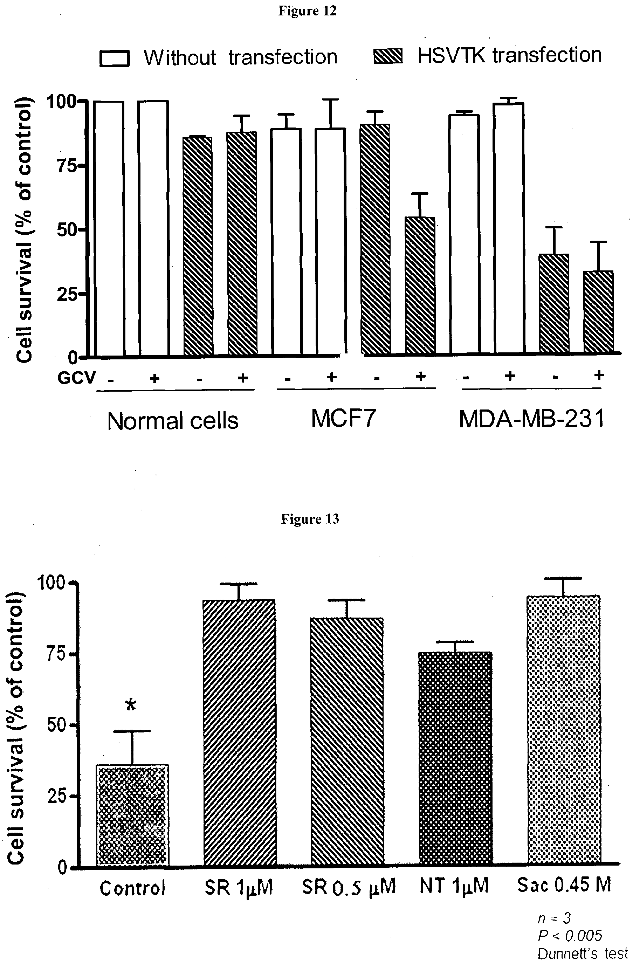

The *in vitro* ability of the cell line MDA-MB-231 derived from breast ductal adenocarcinoma to be transfected by the NTS-polyplex complex is a first approach to antitumor gene therapy for this type of cancer (Figs. 2 and 3). Forgez *et. al.* (2002) found that the breast cancer cell line called MCF7 (from mammary gland adenocarcinoma, as MDA-MB-231) has NTSR1 and NTS3 linking the NTS with a Kd of 2 nM. The cell line MDA-MB-231 represents an extremely invasive cancer. Figure 4 shows the results on an efficient transfection of EGFP reporter gene in the cell lines MDA-MB-231 and MCF7 *in vitro* conditions, while figures 11 and 12 show the transfection *in vivo* conditions.

Theoretically, NTS-polyplex should exert an agonist response on the receptor NTSR1, enabling not only the endocytosis process, but also the signaling cascade. Forgez and col. (2002) report that NTS has an anti-apoptotic effect stimulating NTSR1 and therefore, it would be expected that this phenomenon diminishes the effectiveness of the antitumor strategy mediated by NTS-polyplex. Since gene therapy with NTS-polyplex induces an efficient antitumor effect, it is inferred that the pro-apoptotic effect resulting from the erroneous DNA chain because of the incorporation of active ganciclovir (phosphorylated forms) predominates on the anti-apoptotic effect resulting from the activation of NTSR1. Obviously, the many false bases (active ganciclovir) incorporated into the DNA chain of the malignant cells would produce, first, a defective genome incapable of transmitting malignant inheritance to daughter cells, and secondly, the expression of a large amount of erroneous protein products functionally inefficient. These consequences of suicide therapy are incompatible with the life of the cancer cells and predominate over the anti-apoptotic effect induced by the stimulation of the receptor NTS1. Carraway and Plona (2006) described the presence of the receptor NTS with the following percentages of incidence: 65% in Ewing sarcoma, 52% in meningioma, 43% in astrocytoma, 29% in thyroid cancer, 25% in small cell lung cancer, 5% in breast cancer, 4% in colon cancer, while it is not present in liver, ovarian, or prostate cancer, but occurs in pancreatic adenocarcinoma in 75%. Carraway and Plona (2006) also described that cell lines have a higher incidence of the NTS 1 receptor than the corresponding primary tumors, and that there is also a certain instability of the ligand NTS by the effect of tissue peptidases. The *in vivo* essays that are described in the development of the present invention dispel any doubt on the performance of the internalization of NTS-polyplex and its ability to transfect, for example breast tumor cells. Importantly, breast cancer metastases expressing the receptor NTSR1 are not totally responsive to chemotherapy and radiotherapy; likewise these treatments also produce severe side effects because of their lack of specificity. Therefore, the use of NTS-polyplex of the invention to transfect genetic material as described herein is an excellent therapeutic alternative.

With regard to the possibilities of approaching to genic therapy to treat breast cancer, we can mention the preventive ones, for example:
a) Restoration of occurred mutations in tumor suppressor genes (TSG), for example, because there is a complete loss of the allele and/or there is a dysfunctional allele, TSG are the genes BRCA-1 and BRCA-2 (responsible for approximately 80-90% of hereditary breast cancers); the p53 genes (present in more than 20 to 30% of the cases of spontaneous breast cancer), and/or pRb (present in 30% of breast cancer cases),
b) Suppression of amplified oncogenes;
c) Pro-apoptotic gene therapy;
d)Immunopotentation or genetic modulation (Khalili, Curiel, 2006).

To date, the most frequently amplified genes in breast cancer, called dominant oncogenes, include the epithelial growth-factor c-erbB-2/HER-2 receptor (neu) and c-myc nuclear transcription factor (Bland, KI, 1995).

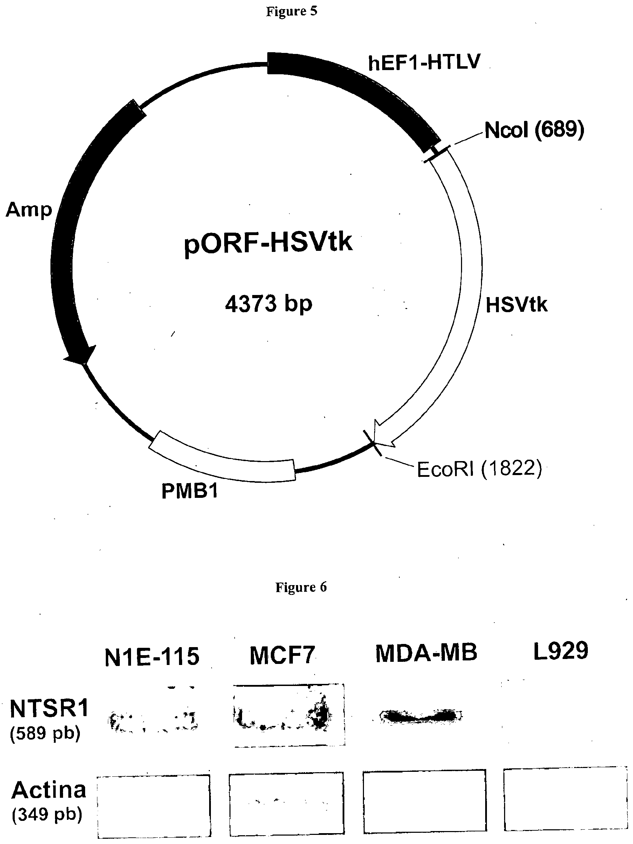

The use of NTS-polyplex complex in molecular chemotherapy protocols as the one described herein is targeted to breast cancer and mesothelioma and comprises the transfection of a suicide gene to a targeted therapy by transfection of an enzyme acting on a prodrug activated (by effect of the enzyme). The suicide gene can be HSV-Tk, and thus the treatment also involves the administration of ganciclovir (GCV), which can be started at 24 hours post-transfection with a continued, therapeutically effective dosage-regimen. With this strategy the transfected enzyme concentration is expected to be higher in the tissue where it is being produced and therefore, only exert there its cytotoxic effect. According with the present invention, figures 5 and 6 shows the results on the efficient transfection of the suicide gene in the cell line MDA-MB-221 at *in vitro* conditions.

The possibilities of the invention extend to the transfection of genes playing a particular or exclusive role of the cells where it is expressed, so that the therapy can be targeted with much greater accuracy to the tumor; and it can be expected that all tumor disappears even without transfecting each one of the cells. So, even in poorly vascularized tumors, which hinder the access of NTS-polyplex, the outlined strategy is very efficient.

The main approach of the invention is proposed as one of the preferred embodiments of the invention, and it refers to the suicide gene transfection. It is a molecular chemotherapy with the best chance of success in eliminating metastatic cells, being a transfection that does not demand the integration of the suicide gen into the genome to express the therapeutic gene. During the development of the present invention, the ability of NTS-polyplex to transfect suicide genes in breast cancer cells was addressed using the murine model, which consists of the xenograft of breast cancer cells in athymic mice, leaving ectopic sites of malignant tumors, and they are therefore, a metastasis model. With this strategy, the removal of tumor is accomplished without exposing the organism to the typical toxicity of conventional chemotherapy as the effects are *in situ.*

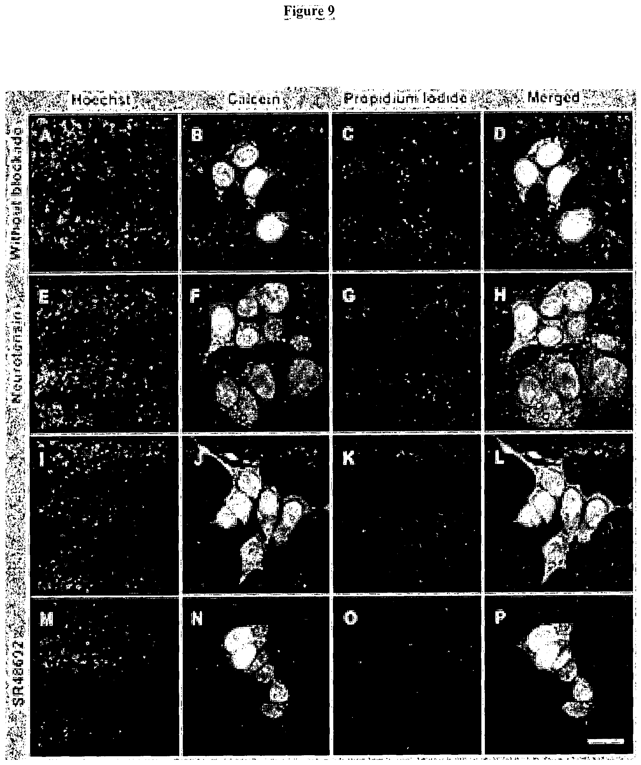

Mice transfected with HSV-TK gene were subjected to treatment with ganciclovir (GCV), having initiated its administration at 24 hours post-transfection and a continued, therapeutically effective dosage-regimen. The results in figure 9 are evidence of the effectiveness of the system of the present invention.

The present invention combines various technical aspects, and presents a strategy designed especially for the treatment of breast cancer by killing cancer cells, including those that are highly proliferative, and reducing the probabilities of occurrence of metastases.

The state of the art reports that the process of receptor-mediated endocytosis is a mechanism that works well for purposes of gene therapy. Even the complex of the NTS-polyplex nanoparticle system can be applied in gene therapy for the mentioned breast cancers, colon and pancreatic cancer, neuroblastoma, mesothelioma, Ewing's sarcoma, astrocytoma, etc. However, before the DNA can be sent to the nucleus and expressed in each of these cases, it is necessary to solve some special difficulties to obtain a successful expression. During the development of the present invention, results were obtained that show the effectiveness of the transfection and the expression of a reporter gene, even a therapeutic gene in the cell lines under study (MFC7, MDA-MB-231) and exposed to NTS-polyplex.

Regarding the expression of the NTSR1 receptor in human cancer cells, in a healthy individual the NTSR1 does not express in most cells of his/her body and is found only in very specific cell and anatomically limited cell populations, such as some nuclei and ganglia of the nervous system and gastrointestinal tract. As a hallmark of cancer, NTSR1 is over-expressed in cells that possessed it physiologically or expressed *de novo* in cells that lacked its expression. In peripheral tissues the NTS is located in high density in the gastrointestinal tract where, in contrast, the receptor NTSR1 is expressed at low levels. In healthy persons, NTSR1 receptor is not expressed in the epithelial cells of breast, colon, lung, and pancreas; but when those epithelial tissues are transformed in solid tumors, the gene encoding NTSR1 receptor is induced to high levels of expression. Therefore, transfection using NTS-polyplex is more effective in tumor cells since they have a higher density of NTS1 receptor than the body's healthy cells.

NTS-polyplex inoculated through the blood stream can transfect tumor cells and healthy cells of the gastrointestinal tract, because NTSR1 is present in these cells, although at different densities. The NTSR1 receptor is expressed at very low levels in the cells of healthy individuals compared to the high density in cancer cells. Therefore, transfection is significantly higher in tumor cells than in the cells of the gastrointestinal tract. The brain, which is the organ with the highest density of NTRS1, is not transfected by NTS-polyplex after its administration through the bloodstream owed to the inability of the NTS to cross the blood-brain barrier. As an advantage of the systemic application of NTS-polyplex in antitumor gene therapy protocols, excels the fact that the central nervous system will be protected from suicide gene transfection (Martinez-Fong *et. al*., 2008).

NTS-polyplex has the advantage of providing an additional point of specificity when using tissue-specific promoters such as hDAT promoter, confining transgenic expression to transfected cells (Martinez-Fong *et. al*., 2006). Healthy cells from peripheral tissues that express the NTSR1 receptor can be protected from the harmful effects of transgene expression using a specific promoter for cancer cells; for example, the promoter of the beta-catenin gen, by that limiting the expression only in malignant cells.

Part of the development of the present invention relates to demonstrating the coexpression of NTSR1 receptor and NTS in a type of highly invasive breast cancer such as the human cell line MDA-MB-231, where the co-expression suggests the functionality of said receptors. This functionality can be extrapolated to the primary tumor tissue and has no precedents in the state of the art.

Also as part of the development of the present invention, it was demonstrated *in vitro* that the activation of the NTSR1 receptor increases some transformant functions of this type of cancer, thus supporting the involvement of NTS through NTSR1 receptor in tumor progression. As expected, expression disruption of NTSR1 receptor using interference RNA technology or pharmacological blockade in nude mice with xenografts of MDA-MB-231 cells decreases tumor progression.

Part of the development of the present invention included the investigation of the molecular mechanisms involved in the activation of NTSR1 receptor in cancer, demonstrating a direct link between the route of Tcf/beta-catenin and the promoter activation of NTSR1 receptor using adenocarcinoma cells of human colon (Bossard, C. *et. al*., 2007). This mechanism is relevant for the control of transgenic expression using beta-catenin synthetic promoter, which is active in cells with high mitotic index, such as tumor cells. Therefore, the use of this promoter is part of one of the embodiments of the invention. However, it can also include other eukaryotic constitutive promoters such as α1 elongation factor promoter.

In a study of ductal breast cancer, the inventors showed that the high expression of NTSR1 receptor correlates with tumor size, the number of metastatic lymph nodes, and the mortality of patients. Ongoing studies show for the first time that NTSR1 receptor is expressed in 80% of patients with mesothelioma and in 65% of patients with non-small lung carcinoma. Therefore, these types of neoplasias and the breast cancer represent a specific target for therapeutic gene transfer via the internalization of NSTR1 receptor using NTS-polyplex.

The efficiency of transfection using NTS-polyplex depends on several factors, including cell type, receptor response and the type of designed signaling pathway, although there is the antecedent of the transfection efficiency when used in neurons and neuroblastomas. With the use of NTS-polyplex in both cell assays *in vitro* and preclinical models, it follows that once NTS-polyplex is internalized by the endosomes, it evades lyososomal degradation, and the genetic material reaches intact the cell nucleus and expresses the gene of interest. It can investigate whether the receptors return to the membrane (recycling) to promote more events of endocytosis and thus increase the chances of transfection.

In theory, one the complex is internalized, it is estimated that it remains in the cytoplasm inside endosomal vesicles from 15 to 30 minutes; however, all consequent events associated with internalization vary depending on the cell type, NTS dose, the type of antagonist or agonist, and the time of exposure.

For example, in Cos-7 cells, co-localization of NTSR1 endosomes with lysosomes has been observed after 45 min exposure to the agonist, so there must be a subsequent degradation. Studies in neuroblastoma cell lines and primary cultures of neurons confirm that the cell loses sensitivity to NTS and sensitizes some time later, once the internalized receptor degradation has occurred, and the *de novo* synthesis happens. Depending on its affinity for beta-arrestine in any of its isoforms a delay occurs in the dephosphorylation of the receptor and slows cellular re-sensitization promoting the receptor to the degradation pathway.

The phenomenon of desensitization of cells exposed to high concentrations of NTSR1 agonists may affect the transfection efficiency of NTS-polyplex during genic therapy. However, this phenomenon occurs at high agonist concentrations, which are not reached by *in vivo* applications, and is a reversible process. *In vitro* studies show that all NTSR1 receptors are reset within 24 hours of continuous exposure to high concentrations of agonist. For this reason, intratumoral or sanguineous administration of NTS-polyplex is done in a single dose that repeats every 48 hrs. Inclusively, NTS-polyplex could be applied in unique dose every 24 hours, which is the time when all NTSR1 receptors have been restored on the cell membrane.

Although the expression of NTSR1 receptor is induced in breast invasive ductal adenocarcinoma, the use of the complex of the present invention is not limited to treatment of breast cancer metastasis stage. It can also be used in certain cases to treat breast carcinoma *in situ*, whether lobular or ductal, and more specifically in ductal carcinoma as a predictor of imminent invasive cancer.

NTS-polyplex can be used to treat invasive or infiltrative ductal carcinoma, which originates in the milk-producing gland, and can spread to the lymph channels or blood vessels of the breast reaching other parts of the body. This is the most common type of tumor in breast cancer, and the NTS-polyplex of the invention can be used to treat medullary carcinoma, which is estimated to be responsible for 5% of all breast cancer cases. In medullary carcinoma, cancer cells are clustered and in the borders of the tumor exist immune system cells that serve to attack and destroy abnormal cells and other foreign agents like bacteria or viruses. The NTS-polyplex of the invention can be used to treat colloid carcinoma, which is formed by mucus-producing cells. This type of ductal cancer is low invasive and has a favorable prognosis, being less likely to spread than the invasive ductal cancer or the invasive lobular cancer.

Within the field of nanotechnology in medicine, the NTS-polyplex of the invention is seen as a system biologically safe for clinical use, with less collateral effects for being more specific and thus more efficient for the treatment of cancers that express the NTSR1 receptor, with special emphasis on mesothelioma treatment and prevention of breast cancer metastasis.

The present invention relates to the ability of the molecular complex called NTS-polyplex administered intravenously to transfect suicide genes in cells of a highly invasive breast cancer.

During the development of the present invention, we performed *in vitro* tests of internalization and expression of reporter genes pEGFP-N1 and pORF-Luc in combination with blocking studies using neurotensin, the specific antagonist SR48692, or sucrose. For functional studies, we used the suicide system pORF-HSVTK-ganciclovir (GCV). Cytoxicity was also evaluated. The RT-PCR and immunofluorescence studies confirmed the presence of NTSR1 in the cell lines tested, which were able to specifically internalize and express the reporter genes. The cell line MDA-MB-231 was more efficiently transfected than the MCF7, but the viability of the MDA-MB-231 cells decreased by 60% during the transfection process.

Regarding the suicide genes, these are part of the preferred embodiments of the invention. During *in vitro* essays with MDA-MB-231 cells, transfection of plasmid pORF-HSVTK decreases cell viability by 50% independently from activation with GCV. Blocking assays suggest that the cytotoxic effect was caused by the efficient endocytosis of NTS-polyplex mediated by NST1. In conclusion, MDA-MB-231 cells were the most susceptible to transfection with NTS-polyplex, which was cytotoxic to this cell line. Since they are highly invasive cells, this effect may represent an additional advantage to achieve a better antitumor therapy for breast cancer.

Using the murine model that consists of practicing xenografts of breast cancer cellular line MDA-MB231 in athymic mice Nu/Nu, we have an animal model in a comparable situation to the invasive cancer by the presence of ectopic sites of primary tumors (metastasis). The metastases treatment of this type of highly invasive breast cancer is the main challenge for oncology, since the primary tumor could be treating with surgery in combination with radiotherapy or chemotherapy.

Some of the most relevant results suggest that apoptosis is the type of cell death mediating the cytotoxic effect of GCV in tumors transfected with HSV-TK suicide gene. In contrast, control tumors showed a large area of necrosis (dark color on the tumor) probably because the blood supply cannot meet the metabolic demands of tumor growth.

The preferred form of administration of NTS-polyplex in the methods of the present invention is the systemic method as it reflects the advantages of the general strategy and can serve for treating metastasic sites difficult to access. However, the administration *in situ* can also be considered for certain cases.

The following examples are representative for obtaining, evaluating, and applying the invention. The results fully support the clinical and experimental uses of NTS-polyplex for the treatment of cancers that are addressed in detail in the description. Because these examples are only included to illustrate the present invention, they are not intended to limit its scope.

### Example 1. Synthesis of NTS-vector: NTS-(PF)-SPDP-PLL.

For the synthesis of the NTS-vector we used following peptides: NTS (sequence ELYENKPRRPYIL), purity > 90% and 1,672 Da molecular mass (MM) (Sigma, St. Louis MO, USA), and PF (sequence GLFEAIAEFIEGGWEGLIEGSAKKK), purity 96% and 2,695 Da of MM (Synpep Corp., Dublin, CA, USA.) Both peptides were simultaneously binded to PLL of MM ranged from 25,600 to 47,900 (average 36,750 Da) (Sigma, St. Louis MO, USA.) This has 251 potentially reactive amino groups. As biofunctional cross-linker we used N-succinimidyl 6-3[3-(2- pyridyldithio) propionamide] hexanoate (LC-SPDP; MM 452.52; Cat. 21651; Pierce Chemical Co, Rockford, IL, USA).

The NTS-vector synthesis is a process that comprises five sequential steps performed at room temperature: 1) formation of PLL-SPDP conjugate; 2) formation of PLL-SPDP-SH conjugate; 3) formation of NTS-SPDP conjugate; 4) formation of PFSPDP; and 5) formation of NTS-vector [NTS-(PF)-SPDP-PLL] from the conjugates previously obtained with SPDP. The PLL (poly-L-lysine) may have a varying molecular weight and in its isomeric form L or D.

### Example 2. Formation of PLL-SPDP-SH conjugate.

25 mg of PLL were dissolved in 2 mL of a phosphate buffer solution (PBS: 17.42 mM de Na₂HPO₄, 2.58 mM KH₂HPO₄, 150 mM NaCl, 1 mM EDTA, pH 7.2). This was mixed with 7.5 mg of LC-SPDP dissolved previously in 30 µL of dimethyl sulfoxide (DMSO). The mixture of PLL with LC-SPDP was incubated for 30 minutes under constant stirring. After this period, the resulting conjugate (PLL-SPDP) was purified on a Econo-Pac 10DG column (Bio-Rad Laboratories, Hercules, CA, USA) equilibrated with PBS. We collected fractions of 1 mL. An aliquot of 3 µl was taken from each fraction, and it was read in a NanoDrop spectrophotometer (NanoDrop Technologies ND_1000) to determine its absorbance at 215 and 280 nm.

Econo-Pac 10DG column allows the elution of < 6,000 Da molecules; thus, the PLL-SPDP conjugate (52,043 Da) is obtained in the first peak (3-7 mL), while free SPDP (425.5 Da) and N-hydroxysuccinimide (114 Da) released as a reaction product elute in the second peak (8-13 mL). Therefore, the fractions corresponding to the first peak were collected and concentrated to a volume of 1 mL, using a vacuum concentrator (Heto). To generate the highly reactive conjugate, the PLL-SPDP-SH and 24 mg of ditiotreitol (DTT) were dissolved in 0.5 mL of PBS and added to 1 mL of the PLL-SPDP solution.

The mixture was incubated during 30 minutes with constant agitation. The PLL-SPDP-SH conjugated was later purified in a Econo CAP 10 DG column balanced with PBS, collecting fractions of 1 ml. An aliquot of 3 µL from each fraction was collected and read in the NanoDrop to determine its absorbance at 215 nm. Considering the rank of separation of this column, the PLL-SPDP-SH conjugated (50,613 Da) elute in the first peak volume (3-6 mL) and pyridine-2-thione (110 Da) elute in the second peak (9-16 mL). The first peak fractions were collected and concentrated at 1 ml.

### Example 3. Formation of NT-SPDP conjugate.

10 mg of NTS were dissolved in 2 mL of PBS and mixed with 5 mg of LC-SPDP dissolved previously in 30 µL of DMSO. The reaction mixture was incubated for 30 minutes under constant stirring. After incubation, the NTS-SPDP conjugate was purified on Sephadex G-10 column (Pharmacia Fine Chemicals AB, Uppsala, Sweden) equilibrated with PBS. Fractions of 0.1 mL were collected; 3 µl aliquots of each fraction were read on NanoDrop to determine their absorbance at 215 and 280 nm. Sephadex G-10 has a circumvention ability of <700 Da; therefore, the NTS-SPDP conjugate (2,419 Da) eluted in the first peak volume (3.5-8.4 mL). Fractions corresponding to the first peak were collected and concentrated at 1 mL.

### Example 4. Formation of PF-SPDP conjugate.

9.2 mg of PF were weighted, diluted in 2 mL of PBS and mixed with 2.5 mg of LC-SPDP, previously dissolved in 30 µL of DMSO. The mixture was incubated during 30 minutes with constant stirring. After this period, the PF-SPDP conjugate was purified on a Sephadex G-15 column (Pharmacia Fine Chemicals AB, Uppsala, Sweden) equilibrated with PBS. We collected 0.1 mL fractions, and 3 µL aliquot of each fraction was read on NanoDrop to determine its absorbance at 280 nm. Chromatographic ability of Sephadex G-15 is < 1,500 Da; therefore, the PF-SPDP conjugate (3,317 Da) eluted in the first peak volume (3.7-7 mL), while the free SPDP (425.5 Da) and the N-hydroxisuccinimide (114 Da) eluted in the second peak (18-31 mL). Fractions corresponding to the first peak were collected and concentrated at 1 mL.

### Example 5. Formation of NTS-vector from the previous conjugates.

The three previously obtained conjugates with SPDP were mixed and incubated for 8 hours under continuous stirring. Finally, the NTS-(PF)-SPDP-PLL was purified on a Biogel A1.5m column (Bio-Rad Laboratories, Hercules, CA, USA) using as a mobile phase a buffer of 2 M guanidine in 10 mM HEPES, pH 7.4. We collected fractions of 1 mL, and aliquots of each fraction were diluted 1:3 with guanidine to measure the absorbance at 215, 280, and 343 nm. Aliquots corresponding to the low-molecular weight conjugate were collected (volume 45 to 65 mL) and reduced to a final volume of 1 mL using a concentrator camera with nitrogen atmosphere (Amicon Corporation, Lexington, MA, USA). At the end of the concentration process, the NTS-vector or carrier of NTS was subjected to successive cell dialysis with PBS (8.1 mM Na₂HPO₄, 1.2 mM KH₂PO₄, 138 mM NaCl, 2.7 Mm KCl, pH 7.4) and sterilized by filtration (hydrophilic membrane of 0.22 mM). The calibration curve was performed with PLL to quantify the NTS-vector and stored at 70°C in small aliquots.

### Example 6. Formation of NTS-polyplex complex.

The NTS-polyplex was assembled maintaining the following proportions: three parts of pDNA (plasmid construct containing the reporter or therapeutic gene), one part of PK and two parts of NTS-vector. The first step is the formation of the pDNA complex. PK from the mutant protein of virus SV40 (MAPTKRKGSCPGAAPNKPK) was synthesized by SynPep (SynPep Corp., Dublin, CA, USA), purity 90%. PK has net positive charge, allowing its electrostatic binding to pDNA (polyanion), to form pDNA-PK-complex. We used constant concentrations of pDNA (6 nM) and PK (5 µM for pEGFP-N1 and 6 µM for pORF-HSVTK).

PK solution is added dropwise to the pDNA solution; the mixture is stirred gently at 900 rpm for 30 minutes. The second step comprises the rapid addition of 1% fetal bovine serum (FBS) to the pDNA-PK solution and finally added dropwise to the solution containing the NTS vector (increasing concentrations of 90 nM to 270 nM) and incubated for 30 minutes with continuous stirring (900 rpm). pDNA, PK, and NTS-vector (NTS-(PF)-SPDP-Poly-lysine conjugated) were individually dissolved in Dulbelcco Modified Eagle medium (DMEM) (Gibco, Invitrogene Co., Grand Island, NY, USA) free of serum. All procedures were performed at room temperature. NTS-vector must be capable of condensing the plasmid DNA into nanoparticles of 50 to 150 nm in diameter (Figure 1) to allow the internalization of the endosome. During the synthesis of the complex, it is considered to obtain precise proportions of DNA maintaining a fixed quantity of DNA and increasing the concentrations of the binding complex to ensure the obtention of a proper ratio with DNA. The molecular weight of the complex is variable.

### Example 7. Plasmids.

NTS-polyplex can transfect different plasmids to cells. Therefore, in the different experiments we used plasmids whose restriction maps are illustrated in figures 2 to 5; plasmid pEGFP-N1 (4.7 Kbp encodes green fluorescent protein (GFP) and is under the control of the cytomegalovirus promoter (CMV) (Clontech, Palo Alto, CA, USA). Plasmid pTracer-EF/V5-His (5.93 Kbp) encoding GFP is under the control of the CMV promoter; it further has the EF-α promoter that provides high level expression in mammalian cell lines and controls the expression of a gene of interest (Invitrogen, Carlsbad, CA, USA).

Plasmid pORF-LUc (4.896 Kpb) encoding luciferase enzyme (Luc) was obtained from cloning cDNA Luc (1.656 Kpb) in the sense orientation Nco1-Nhe1 of the pORF expression vector; the cDNA of Luc was obtained previously from pGL3-Basic Vector (Promega Corporation, Madison, WI, USA) using enzymes Nco1 and Xba1.

Plasmid pORF-HSVTK (4.373 Kbp) encodes thymidine kinase enzyme (TK) of HSV, and is under a hybrid promoter composed of alpha-1 elongation factor promoter and 5' untranslated region of leukemia virus of human T cells (Invivogen, San Diego, CA, USA).

### Example 8. NTSR1 detection in MDA-MB-231 and MCF7 breast cancer cell lines.

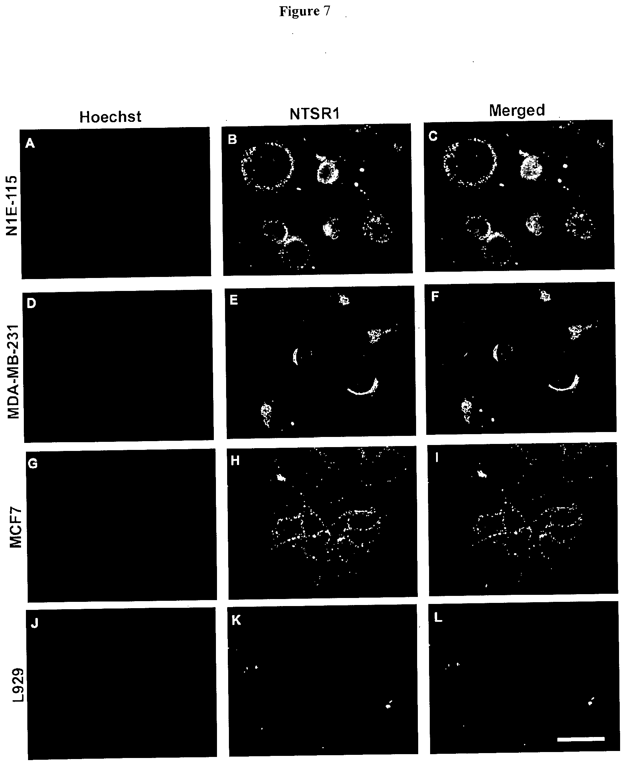

The presence of NSTR1 in MDA-MB-231 and MCF7 breast cancer cell lines was demonstrated by RT-PCR studies (Fig. 6) and immunofluorescence (Fig. 7). For the PCR reaction we used specific primers for NTSR1 and for actin. The RT-PCR showed a band of 589 bp corresponding for NTSR1 in the N1E-115 cell line (positive control) and in both breast cancer lines. In contrast, this band was absent in L929 cell line (negative control). In all three lines, we observed the band of 349 bp corresponding to actin amplicon. In turn, the presence of NTSR1 was also confirmed by indirect immunofluorescence essay. Each of the lines N1E-115, MDA-MB-231 and MCF7 showed a differential distribution pattern of the receptor. The control line N1E-155 showed a dotted mark corresponding to a membrane or submembranal location. In cell line MDA-MB-231 the mark was preferably located in the perinuclear region, while in the cell line MCF7 the distribution pattern corresponds to the cell membrane (chicken wire) characteristic of cells with epithelial morphology. By contrast, we did not observe immunoreactivity in L929 cell line.

### Example 9. Internalization of the NTS-polyplex of the invention in the MDA-MB-231 and MCF7 cell lines via NTSR1 endocytosis.

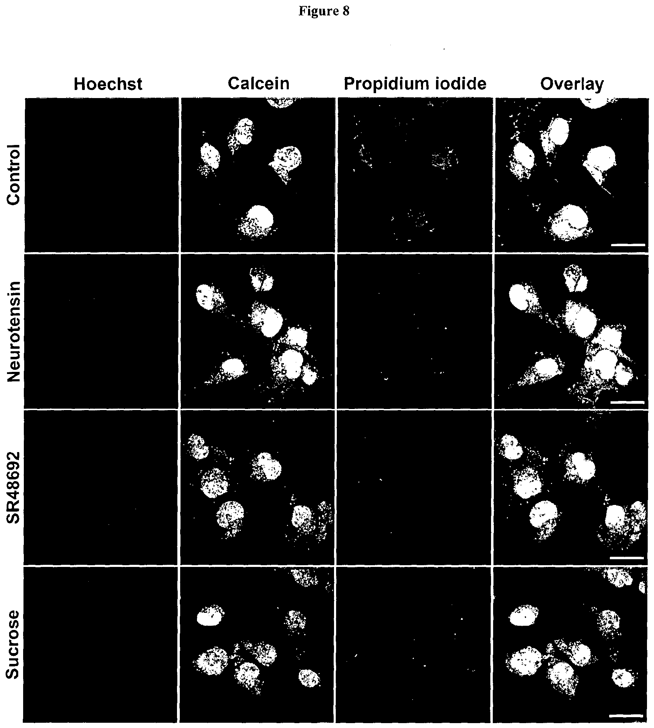

The functionality of NTSR1 to internalize via endocytosis the NTS-polyplex was evaluated in the breast cancer cell lines using blocking assays with NTS (1 µM), SR48692 (500 nM), or sucrose (0.45 M). The pDNA of NTS-polyplex was marked with propidium iodide (red) to reveal its location in the nucleus stained Hoechst 33258 (blue). The cytoplasm was stained with calcein AM (green). Cells were analyzed by confocal multi spectral imaging system TCS-SPE (Leica Mycrosystems, Wetzlar, Germany). The fluorescence was detected under the following conditions Ex/Em: 405/430 nm for the blue channel with UV light; 488/512-573 nm for the green channel with an argon laser; and 563/563-544 for the red channel with a Helium-Neon laser. We obtained 10 to 20 consecutive optical sections of 1 µm apart in the z series. MDA-MB-231 cells (Fig. 8) showed increased fluorescence of propidium iodide in the nucleus of MCF7 cells (Fig. 9), suggesting that they are more efficient to internalize NTS-polyplex.

As expected, blocking the NTSR1 binding site with NTS or SR48692 prevented the entrance of NTS-polyplex in both cell lines (Figs. 8 and 9). The internalization of NTS-polyplex was also blocked by inhibiting the formation of endosomes by incubation with the hypertonic solution of sucrose (Figs. 8 and 9). These results confirmed the specificity of gene transfer by NTS-polyplex through NTSR1 endocytosis.

### Example 10. GFP reporter gene expression in MDA-MB-231 and MCF7 cells transfected with the NTS-polyplex of the invention.

Specific internalization of pEGFP-N1 plasmid in MCF-7 and MDA-MB-231 cells by NTS-polyplex leads to GFP reporter gene expression (Fig. 10). The cells were incubated with NTS-polyplex formed at the molar concentration that produced the highest efficiency of internalization, which was pEGFP-N1 (6 nM), PK (5 µM), and NTS-vector (180 nM). Forty-eight hours post-transfection the cells were fixed with 4% paraformaldehyde, washed with PBS and fixed with 1 mM Hoechst 33258. The fluorescence in cells was analyzed with a 20X objective of DMIRE2 Leica microscope (Leica Microsystems, Wetzlar, Germany) using the following filters: A for Hoechst 33258 and K3 for GFP. Images were digitized with a Leica DC3005 camera (Leica Mycrosystems; Nussloch, Germany).

### Example 11. Early-onset of apoptosis by transfection of HSVTK suicide gene in MDA-MB-231 cells using the NTS-polyplex of the invention.

Detection of phosphatidylserine translocation by Annexin V-FITC, a marker of early apoptosis, more specifically showed the damaged caused by the transfection of plasmid pORF-HSVTK in MDA-MB-231 cells. At 24 hours post transfection, the fluorescence of Annexin V was observed only in cells transfected with NTS-polyplex at optical 1:30 molar ratio. In contrast, there was no mark at ratio 1:15 (ineffective doses) (Fig. 11). Because cells were stained with propidium iodide after fixation, this assay did not allow the evaluation of the participation of necrosis as another possible mechanism of cell death. An apoDETECT ANNEXIN V-FITC kit was used to demonstrate the phosphatidylserine (PS) traslocation in the outer membrane of the cell membrane (Fig. 11, panels A and D). Cells were stained with propidium iodide (Fig. 11, panels B to E). Panels C and F of figure 11 correspond to the images of the same line. The photomicrographs represent two independent experiments. The fluorescence was observed with a Leica DMIRE2 microscope with a K3 filter for FITC and a TX2 filter for propidium iodide.

### Example 12. Cell density decrease after transfection of HSVTK suicide gene using the NTS-polyplex of the invention.

The breast cancer cell lines MDA-MB-231 and MCF7, and primary cultures derived from healthy breast tissue (negative control) were exposed to NTS-polyplex containing plasmid pORF-HSVTK at optimum molar ratio (1:30). Twenty-four hours later, the transfection medium was removed and culture medium was added, supplemented with BFS at 10% and GCV 10 µg/ mL. This medium with GCV was renewed every 24 hours for two days. The cytotoxic effect of HSVTK-GCV system was demonstrated by colorimetric MTT cell viability test. The activation of suicide system with GCV did not affect cell feasibility of healthy human cells, but decreased over 50% the cell feasibility of cancer lines MDA-MB-231 and MCF7. Interestingly, the single transfection of NTS-polyplex drastically decreased cell feasibility of MDA-MB-231 line (Fig. 12). This effect is related to the great ability of these cells to endocyte NTS-polyplex, as demonstrated in example 9.

### Example 13. Inhibition of the toxicity of transfection with the NTS-polyplex of the invention in MDA-MB-231 cells by blocking activation of NTSR1.

MDA-MB-231 cells were incubated under various blocking conditions for 30 minutes before the addition of NTS-polyplex containing plasmid pORF-HSVTK, except for the positive control. After this period, NTS-polyplex was added, previously stained with propidium iodide (10 mM) and formed at its optimal molar ratio for pORF-HSVTK (1:30). At six hours post-transfection, GCV 10 µg/ mL was added. Twenty-four hours post-transfection, culture medium was removed and medium supplemented with BFS at 10% and GCV 10 µg/ mL was added. This GCV medium was renewed every 24 hours for two days. Forty-eight hours post-transfection, cell feasibility was assessed by MTT colorimetric test. NTSR1, NTS (1 µM) or SR48692 (0.5 µM or 1 µM) competitive blockers prevented the cytotoxic effect of the interaction between NTS-polyplex and NTSR1 in the presence of GCV (Fig. 13). Furthermore, this cytotoxic effect was prevented by blocking endocytosis by incubation in 0.45 M sucrose (Fig. 13). Together, blocking-tests confirm that the toxic effect resulting from the transfection of NTS-polyplex in MDA-MB-231 cell line, produced by the interaction between NTS-polyplex and NTSR1 or receptor mediated endocytosis. Therefore, massive but specific endocytosis alone would provide some reduction in the number of malignant cells *in vivo.* This is included here as an additional contribution of the invention.

### Example 14. The animal model of breast cancer.

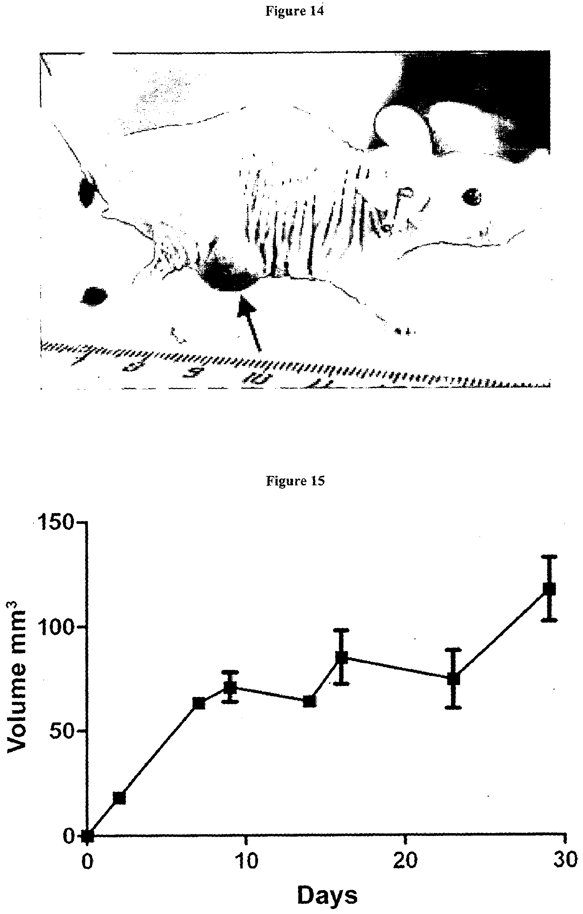

Assays were performed in athymic 4-wk old female Nu/Nu mice, which were inoculated with 3 x 10⁶ MDA-MB-231 cells in the subcutaneous layer of the right flank (Fig. 14). For internalization tests, cells expressing cloned NTSR1 coupled to GFP were xenotransplanted, while for expression tests, wild lines cells were used. The animals were maintained at 23°C in sterile boxes with food and water *ad libitum,* with light and dark periods of 12-12 hours; they were monitored and the progressive growth of the tumor was recorded (Fig. 15). To prevent the progressive tumor growth from interfering with vital functions of the animals and minimize suffering, the study was completed at 3 weeks after the cell line graft. At that time the tumors had an average volume of 75 mm³ (Fig. 15). All procedures were performed in agreement with Mexican legislation (NOM-062-ZOO-1999; SAGARPA) based on the NRC guide for care and use of laboratory animals. The committee for the use and care of animals of CINVESTAV oversaw and approved our experimental procedures.

### Example 15. NTS-polyplex internalization of the invention in vivo.

Plasmid pEGFP-N1 was labeled with propidium iodide (10 mM) before forming the polyplex of NT, as reported previously (Martinez-Fong, 2000).

The marked polyplex (300 µL) was inoculated into the tumor at a rate of 10 µL/min through an infusion micro pump (Bionanalytical System, Inc. Model MD 1001, West Lafayette IN, USA). Six hours after inoculation, under deep anesthesia the animals were transcardiacally perfused with 50 mL of phosphate saline buffer (PBS) pH 7.4, and then with 4% PFH. We proceeded to dissect the tumor and then keep them in 30% sucrose as cryoprotection during 36 hours. Subsequently, 12 µm thick cross-sections were performed. The slices were mounted on slides and covered with ProLong Gold (Invitrogen) to protect the fluorescence. The fluorescence of the cells and the NT-polyplex were observed in a TCS-SPE confocal multi spectral imaging system (Leica Microsystems, Wetzlar, Germany). The fluorescence was detected under following Ex/Em conditions: 488/512-573 nm for the green channel with an argon laser, and 563/563-544 for the red channel with a Helium-Neon laser. We obtained 10 to 20 consecutive optical sections of 1 µm apart in z series.

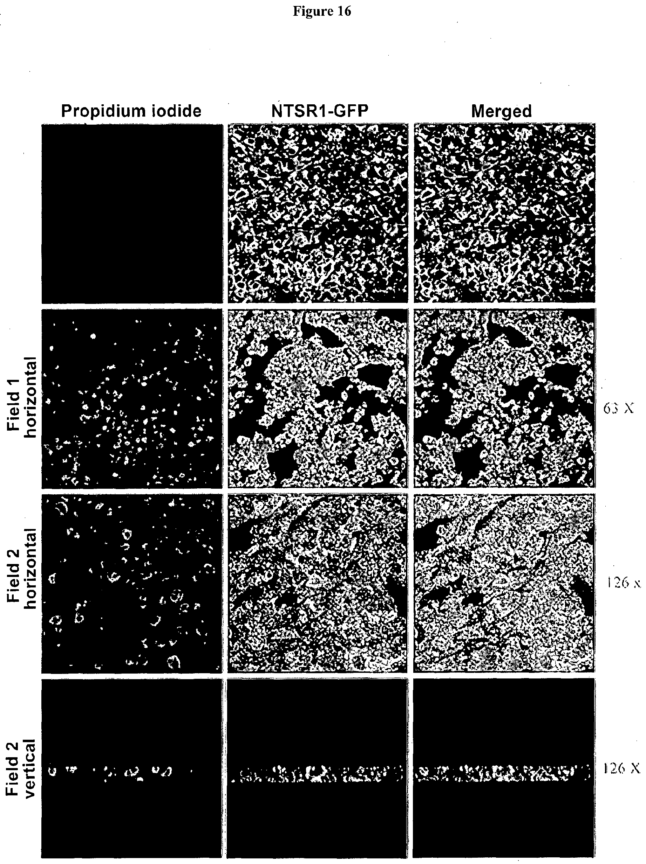

The horizontal plane of confocal cut sections showed the red fluorescent mark of NTS-polyplex inside MDA-MB-231 cells genetically modified to express NTSR1-GFP. Similar results were obtained in the vertical plane of confocal sections. These results demonstrate the ability of MDA-MB-231 to internalize the NTS-polyplex of the invention *in vivo* (Fig. 16).

### Example 16. Transgene expression in vivo.

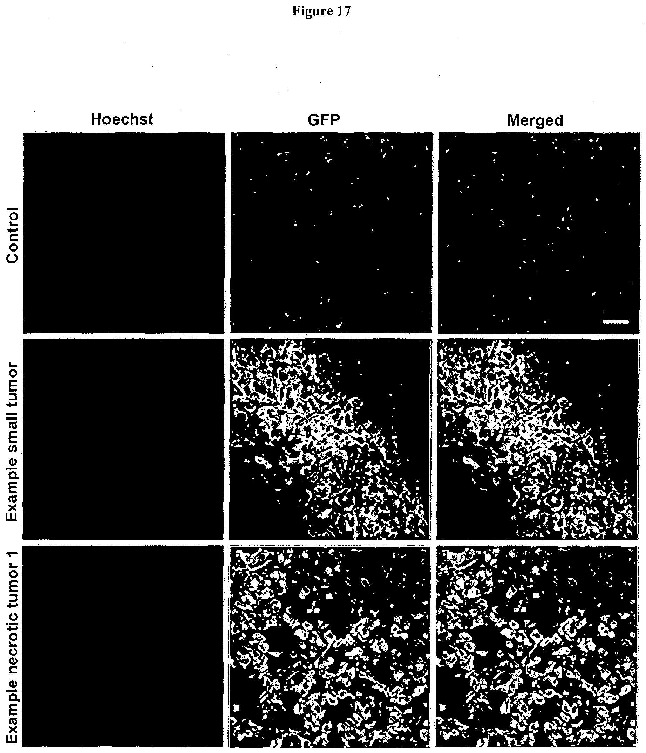

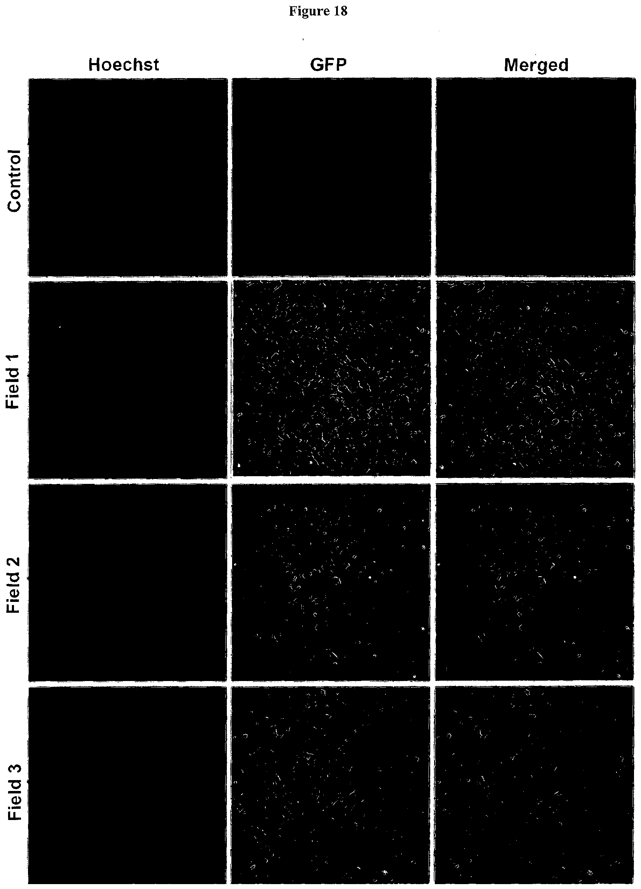

The polyplex with plasmid pEGFP-N1 (300 µL), which allows the expression of fluorescent green protein, or the controls were inoculated into the tumor at a rate of 10 µL/min with an infusion micro pump (Bionanalytical System, Inc., Model MD 1001, West Lafayette, IN, USA). Three days later, under deep anesthesia, the animals were transcardiacally perfused with 50 mL of phosphate saline buffer (PBS) pH 7.4 and then with 4% PFH. We proceeded to dissect the tumor and then kept it in 30% sucrose as cryoprotection for 36 hours. Subsequently, 12 µm thick cross-sections were performed, and the slices were counterstained with Hoechst 33258 (Sigma Co., Saint Louis, MO, USA). The slices were mounted on slides and covered with ProLong Gold (Invitrogen) to protect the fluorescence. The fluorescence of the cells and the NT-polyplex were observed in a TCS-SPE confocal multi spectral imaging system (Leica Microsystems, Wetzlar, Germany). The fluorescence was detected under following Ex/Em conditions: 488/512-573 nm for the green channel with an argon laser, and 405/430 nm for the blue channel with UV light. As expected, the internalization of the NTS-polyplex of the invention *in vivo* also led to the GFP expression in MDA-MB-231 tumor cells identified with nuclear stain Hoechst 33258. The expression was efficient and extensive as shown by confocal microscopy studies (Figure 17). Consistent with intratumoral transfection, the polyplex with plasmid pEGFP-N1 (300 µL) inoculated in bolus into the retro-ocular venous sinus (systemic transfection) also led to GFP expression by MDA-MB-231 tumor cells *in vivo* (Fig. 18). This expression was documented by multi spectral confocal microscopy three days post-transfection intravenously, in 12 µm thick cross sections and counterstained with Hoechst 33258 (Sigma Co., Saint Louis, MO, USA), using the conditions described for intratumoral transfection.

### Example 17. Volume decrease, growing rate, and mass of the tumor after transfection of the suicide gene pORF-HSVTK using the NTS-poliplex.

The experiments were made on Nu/Nu athymic female mice age 4 weeks. They were inoculated with 3 x 10⁶ of MDA-MB-231 cells in the subcutaneous right flank (figure 14). Tumor-growing rate was evaluated progressively until reaching 100 mm³, and then the animals were separated into three groups: a control group without treatment, a group treated via intratumor transfection, and a group transfected via blood stream. The NTS-polyplex harboring the plasmid pORF-HSVTK coding for HSVTK suicide gene (300 µL) was used for transfections. For transfections into tumors, the animals were deeply anesthetized with a mixture of ketamine-xylazine (5:1 mg / Kg of body weight, i.p.; Pisa Agropecuaria, SA de CV, Mexico). A micropump (model MD1001; Bioanalytical System Inc., West Lafayette, IN) was used to inject 300 µl of NT-polyplex into a tumor at a flow rate of 10 µl / min. For intravenous transfections, 300 µl of NTS-polyplex solution was injected in a bolus dose into the retro-ophthalmic vein using an insulin syringe (27 g 13 mm). The GCV treatment (100 mg / Kg of body weight, i.p.) was begun 24 h after the transfection and repeated daily. On completion of the experiments, the animals were killed under deep anesthesia, perfused transcardially with 50 ml of PBS and then fixed with 50 ml of 4% paraformaldehyde. The tumors were sectioned, and documented with photographs and weighted. Finally, the tissue was preserved in 4% paraformaldehyde. All the procedures were in accordance with the Mexican legislation (NOM-062-ZOO-1999; SAGARPA) based on the Guide for the care and use of laboratory animals, NRC. The CINVESTAV Committee for animal care and use (IACUC) approved and supervised our experimental procedures.

The results obtained (Fig. 19) clearly show that NTS-polyplex is able to transfect a suicide gene into tumorous cells derived from the human breast cancer cell line MDA-MB-231 and induce a therapeutic effect. The most striking feature is the suicide effect caused by the intravenous injection of NTS-polyplex, which is a nanoparticle system for a targeted gene delivery.

The targeted gene therapy mediated by NTS-polyplex of the invention has more advantages than viral transductions because viral vectors are not specific when intravenously injected. Based on the findings that NTS-polyplex is a safe delivery system, its use holds great promise for breast cancer treatment in humans.

### References.

1. Pass, I., Vogelzang, N., Carbone, M. Malignant Mesothelioma: Advances in Pathogenesis, Diagnosis, and Transitional Therapies. Springer: New York. (2005).
2. Martinez-Fong, D. et.al. Vector fusogénico y cariofilico para transferencias génicas mediadas por receptor y usos consecuentes. CINVESTAV-IPN. México.MX 264932B, (2001).
3. Martinez-Fong, D. et. al. Improved neurotensin-vector-mediated gene transfer by the coupling of hemagglutinin HA2 fusogenic peptide and Vp1 SV40 nuclear localization signal. Brain research. Molecular brain research, (2002);105(1-2):86-97.
4. Martinez-Fong, D. et. al. Biophysical characteristics of neurotensin polyplex for in vitro and in vivo gene transfection. Biochimica et biophysica acta (2006);1760(7):1009-20.
5. Wu, G. *et al*., Targeted delivery of genes encoding secretory proteins. targeted delivery of genes encoding secretory proteins. EP0587738B. University of Connecticut, (2000).
6. Tatcher, D.R. et. al. Compositions for insulin-receptor mediated nucleic acid delivery. US 5830852, Cobra Therapeutics, Ltd. (London, GB), (1998).
7. Chen, J. et. al. A novel gene delivery system using EGF receptor-mediated endocytosis. FEBS Letters. (1994); 338(2) : 167-169.
8. Midoux P et al. Specific gene transfer mediated by lactosylated poly-L-lysine into hepatoma cells. Nucleic Acids Res.(1993); 21:871-878.
9. Rubio-Zapata, HA, Rembao-Bojorquez JD, Arango-Rodriguez ML, Dupouy S, Forgez P, Martinez-Fong D. NT-polyplex: a new tool for therapeutic gene delivery to neuroblastoma tumors.Cancer Gene Ther. (2009) Jul;16(7):573-84.
10. Martinez-Fong D, et.al. In vivo gene transfer to dopamine neurons of rat substantia nigra via the high-affinity neurotensin receptor. Molecular medicine (2001); Cambridge, Mass; 7(3):186-92.
11. Forgez P. , et .al. Neurotensin counteracts apoptosis in breast cancer cells Biochemical and Biophysical Research Communications. (2002), 295(2):482-488.
12. Forgez P, et. al. Expression of neurotensin and NT1 receptor in human breast cancer: a potential role in tumor progression. Cancer Res. (2006), 66(12):6243-9.
13. Carraway RE, Plona AM. Involvement of neurotensin in cancer growth: evidence, mechanisms and development of diagnostic tools. Peptides. (2006), 27(10):2445-60.
14. Khalili, Curiel , 2006. Gene therapy for carcinoma of the breast. Cancer Gene Ther. (2006) 13(7): 633-647.
15. Bland KI, Konstadoulaki MM, Vezeridis MP, Wanebo HJ. Oncogene protein co-expression: Value of Ha-ras, c-myc, c-fos, and p53 as prognostic discriminants for breast carcinoma. Ann. Surg. 221 (1995), pp. 706-720.
16. Forguez, P. et al. PCT/EP2009/067094 Methods for the treatment and the prognostic assessment of malignant pleural mesothelioma. INSERM. WO/2010/069929. (2010).
17. Forgez P et. al.Neurotensin expression and outcome of malignant pleural mesothelioma. Biochimie, (2010), 92(2):164-170.
18. Bossard C, Souaze F, Jarry A et al. Over-expression of neurotensin high-affinity receptor 1 (NTS1) in relation with its ligand neurotensin (NT) and nuclear beta-catenin in inflammatory bowel disease-related oncogenesis. Peptides (2007), 28: 2030-5.
19. Frédérique Souazé y Patricia Forgez. Molecular and cellular regulation of neurotensin receptor under acute and chronic agonist stimulation. Peptides, (2006), 27(19):2493-2501.
20. Salmons B, Brandtner EM, Hettrich K, Wagenknecht W, Volkert B, Fischer S, Dangerfield JA, Gunzburg WH. Encapsulated cells to focus the metabolic activation of anticancer drugs. Curr Opin Mol Ther. 2010.
21. Lipinski KS, Djeha HA, Gawn J, Cliffe S, Maitland NJ, Palmer DH, Mountain A, Irvine AS, Wrighton CJ. Optimization of a synthetic beta-catenin-dependent promoter for tumor-specific cancer gene therapy. Mol Ther. 2004 Jul;10(1):150-161.
22. Patyar S, Joshi R, Byrav DS, Prakash A, Medhi B, Das BK. Bacteria in cancer therapy: a novel experimental strategy. J Biomed Sci. 2010 Mar 23;17(1):21. Review.

## Claims

1. A NTS-polyplex molecular complex, capable of transferring genes *in vitro* or *in vivo* into breast cancer cells that have NTSR1 receptor on their surface through its receptor-mediated endocytosis, comprising :
a) A plasmid carrying the gene to be transfected into cancer cells;
b) A caryophillic peptide electrostatically binded to a);
c) Poly-L-lysine also electrostatically binded to a);
d) Neurotensin chemically coupled to poly-L-lysine;
e) At least one fusogenic peptide chemically coupled to poly-L-lysine; and
f) Alternatively includes a tissue-specific transcriptional promoter, wherein said fusogenic peptide chemically coupled to poly-L-lysine consists of the following sequence GLFEAIAEFIEGGWEGLIEGSAKKK.

2. The molecular complex NTS-polyplex of claim 1, wherein the plasmid being transferred carries a suicide gene, which is transfected into cancer cells.

3. The molecular complex NTS-polyplex of claim 2, wherein the suicide gene encodes HSV-Tk enzyme.

4. The molecular complex NTS-polyplex of claim 1, wherein the plasmid being transferred carries a suicide gene, which is an apoptosis inductor protein activating other downstream proteins selected from the group consisting of GSK3, Bax/Bc12, Bac/Bc12, or caspases.

5. The molecular complex NTS-polyplex of claims 1 to 3, wherein the gene being transfected is regulated by a hybrid promoter composed of alpha-1 elongation factor promoter and 5' untranslated region of leukemia virus of human T cells.

6. The molecular complex of claims 1 to 5, wherein the poly-L-lysine can be of varying molecular weight and being in its isomeric form L

7. The molecular complex of claims 1 to 6 for use in the treatment of breast cancer or cancers expressing functional NTSR1 receptor such as mesothelioma, colon cancer, lung cancer, pancreatic cancer, prostate cancer, or Ewing's sarcoma.

8. The molecular complex for use according to claim 7, wherein breast cancer is breast ductal adenocarcinoma, pre-invasive or invasive, either to prevent or treat metastatic events.

9. The molecular complex for use according to claim 7 or 8 administered in conjunction with a pro-drug substance, such as GCV, following a chemotherapy protocol specially designed, in therapeutically effective doses at particular times.

10. The molecular complex for use according to claims 7 to 9 for systemic, intravenous, or *in situ* administration.

11. The molecular complex for use according to claims 7 to 10 , wherein the treatment comprises conventional radiotherapy and chemotherapy.

12. The use of the molecular complex of one of the claims 1 to 6, to transfect *in vitro* cells having functional NTS1.

13. A pharmaceutical composition for use in the treatment of cancer cells that have NTSR1 receptor on their surface, comprising the NTS-polyplex molecular complex of the claims 1 to 6 and an acceptable pharmaceutical vehicle.

14. The pharmaceutical composition for use according to13, comprising the NTS-polyplex molecular complex of the claim 2.

15. The pharmaceutical composition for use according toclaim 13, characterized because comprise the NTS-polyplex molecular complex of the claim 3.

16. A fusogenic peptide consisting of the sequence GLFEAIAEFIEGGWEGLIEGSAKKK.

## Patentansprüche

1. NTS-Polyplex-Molekularkomplex, der geeignet ist, Gene *in vitro* oder *in vivo* in Brustkrebszellen zu übertragen, die NTSR1-Rezeptor auf ihrer Oberfläche durch ihre rezeptorvermittelte Endozytose aufweisen, umfassend:
a) Ein Plasmid, das das zu transfizierende Gen in die Krebszellen trägt;
b) ein karyophiles Peptid, das elektrostatisch an a) gebunden ist;
c) Poly-L-Lysin, das ebenfalls elektrostatisch an a) gebunden ist;
d) Neurotensin, das chemisch an Poly-L-Lysin gebunden ist;
e) wenigstens ein fusogenes Peptid, das chemisch an Poly-L-Lysin gebunden ist; und
f) alternativ mit einem gewebespezifischen transkriptionalen Promoter, wobei das fusogene Peptid, das chemisch an Poly-L-Lysin gebunden ist, die folgende Sequenz umfasst GLFEAIAEFIEGGWEGLIEGSAKKK.

2. NTS-Polyplex-Molekularkomplex nach Anspruch 1, wobei das übertragene Plasmid ein Suizidgen trägt, das in Krebszellen transfiziert wird.

3. NTS-Polyplex-Molekularkomplex nach Anspruch 2, wobei das Suizidgen ein HSV-Tk-Enzym kodiert.

4. NTS-Polyplex-Molekularkomplex nach Anspruch 1, wobei das übertragene Plasmid ein Suizidgen trägt, das ein Apoptosis-Induktorprotein ist, das andere Downstream-Proteine aktiviert, die aus der Gruppe ausgewählt werden, die aus GSK3, Bax/Bc12, Bac/Bc12 oder Caspasen besteht.

5. NTS-Polyplex-Molekularkomplex nach den Ansprüchen 1 bis 3, wobei das transfizierte Gen durch einen Hybridpromoter reguliert wird, der aus Alpha-1-Elongation-Faktor-Promoter und 5' untranslatierter Bereich des Leukämievirus von menschlichen Zellen zusammengesetzt ist.

6. Molekularkomplex nach den Ansprüchen 1 bis 5, wobei das Poly-L-Lysen variierendes Molekulargewicht aufweisen kann und in seiner isomeren Form L vorliegt.

7. Molekularkomplex nach den Ansprüchen 1 bis 6 zur Verwendung bei der Behandlung von Brustkrebs oder Krebsarten, die einen funktionalen NTSR1-Rezeptor ausdrücken, wie zum Beispiel ein Mesotheliom, Darmkrebs, Lungenkrebs, Bauchspeicheldrüsenkrebs, Prostatakrebs oder Ewing's Sarkom.

8. Molekularkomplex zur Verwendung nach Anspruch 7, wobei Brustkrebs ein duktales Brust-Adenokarzinom ist, präinvasiv oder invasiv, entweder zum Verhindern oder Behandeln von Metastasevorkommen.

9. Molekularkomplex zur Verwendung nach Anspruch 7 oder 8, der in Verbindung mit einer Proding-Substanz verabreicht wird, wie zum Beispiel GCV, gemäß einem Chemotherapie-Protokoll, das speziell ausgelegt ist, in therapeutisch wirksamen Dosen zu bestimmten Zeiten.

10. Molekularkomplex zur Verwendung nach den Ansprüchen 7 bis 9 zur systemischen, intravenösen oder *in situ*-Verabreichung.

11. Molekularkomplex zur Verwendung nach den Ansprüchen 7 bis 10, wobei die Behandlung eine konventionelle Strahlentherapie oder Chemotherapie umfasst.

12. Verwendung des Molekularkomplexes nach einem der Ansprüche 1 bis 6, um *in vitro*-Zellen zu transfizieren, die funktionales NTSR1 aufweisen.

13. Pharmazeutische Zusammensetzung zur Verwendung bei der Behandlung von Krebszellen, die NTSR1-Rezeptor auf ihrer Oberfläche aufweisen, umfassend den NTS-Polyplex-Molekularkomplex nach den Ansprüchen 1 bis 6 und einen annehmbaren pharmazeutischen Trägerstoff.

14. Pharmazeutische Zusammensetzung zur Verwendung nach Anspruch 13, die den NTS-Polyplex-Molekularkomplex nach Anspruch 2 umfasst.

15. Pharmazeutische Zusammensetzung zur Verwendung nach Anspruch 13, **dadurch gekennzeichnet, dass** sie den NTS Polyplex-Molekularkomplex nach Anspruch 3 umfasst.

16. Fusogenes Peptid, das aus der Sequenz GLFEAIAEFIEGGWEGLIEGSAKKK besteht.

## Revendications

1. Complexe moléculaire NTS-polyplex, capable de transférer des gènes *in vitro* ou *in vivo* dans des cellules de cancer du sein qui comportent un récepteur NTSR1 sur leur surface par l'intermédiaire de son endocytose médiée par le récepteur, comprenant ;
a) un plasmide portant le gène à transfecter dans les cellules cancéreuses ;
b) un peptide caryophile lié de façon électrostatique à a) ;
c) de la poly-L-lysine également liée de façon électrostatique à a) ;
d) de la neurotensine couplée chimiquement à la poly-L-lysine ;
e) au moins un peptide fusogène couplé chimiquement à la poly-L-lysine ; et
f) comprend en variante un promoteur de la transcription spécifique du tissu, dans lequel ledit peptide fusogène couplé chimiquement à la poly-L-lysine est constitué de la séquence suivante GLFEAIAEFIEGGWEGLIEGSAKKK.

2. Complexe moléculaire NTS-polyplex selon la revendication 1, dans lequel le plasmide transféré porte un gène suicide, qui est transfecté dans les cellules cancéreuses.

3. Complexe moléculaire NTS-polyplex selon la revendication 2, dans lequel le gène suicide code pour l'enzyme HSV-Tk.

4. Complexe moléculaire NTS-polyplex selon la revendication 1, dans lequel le plasmide transféré porte un gène suicide, qui est une protéine inductrice d'apoptose activant d'autres protéines en aval choisies dans le groupe constitué de GSK3, Bax/Bcl2, Bac/Bcl2, ou de caspases.

5. Complexe moléculaire NTS-polyplex selon les revendications 1 à 3, dans lequel le gène transfecté est régulé par un promoteur hybride composé du promoteur du facteur d'élongation alpha-1 et de la région non traduite en 5' du virus de la leucémie des lymphocytes T humains.

6. Complexe moléculaire selon les revendications 1 à 5, dans lequel la poly-L-lysine peut être de poids moléculaire variable et est sous sa forme isomère L.

7. Complexe moléculaire selon les revendications 1 à 6 pour une utilisation dans le traitement du cancer du sein ou de cancers exprimant un récepteur NTSR1 fonctionnel tels que le mésothéliome, le cancer du côlon, le cancer du poumon, le cancer pancréatique, le cancer de la prostate, ou le sarcome de Ewing.

8. Complexe moléculaire pour une utilisation selon la revendication 7, le cancer du sein étant un adénocarcinome canalaire du sein, pré-invasif ou invasif, pour soit traiter soit prévenir des événements métastatiques.

9. Complexe moléculaire pour une utilisation selon la revendication 7 ou 8, administré conjointement avec une substance précurseur, telle que le GCV, à la suite d'un protocole de chimiothérapie conçu spécialement, dans des doses thérapeutiquement efficaces à des moments particuliers.

10. Complexe moléculaire pour une utilisation selon les revendications 7 à 9, pour une administration systémique, intraveineuse, ou *in situ.*

11. Complexe moléculaire pour une utilisation selon les revendications 7 à 10, le traitement comprenant une radiothérapie et une chimiothérapie traditionnelles.

12. Utilisation du complexe moléculaire selon les revendications 1 à 6, pour transfecter *in vitro* des cellules comportant un NTSR1 fonctionnel.

13. Composition pharmaceutique pour une utilisation dans le traitement des cellules cancéreuses qui comportent un récepteur NTSR1 sur leur surface, comprenant le complexe moléculaire NTS-polyplex selon les revendications 1 à 6 et un véhicule pharmaceutique acceptable.

14. Composition pharmaceutique pour une utilisation selon la revendication 13, comprenant le complexe moléculaire NTS-polyplex selon la revendication 2.

15. Composition pharmaceutique pour une utilisation selon la revendication 13, **caractérisée en ce qu'**elle comprend le complexe moléculaire NTS-polyplex selon la revendication 3.

16. Peptide fusogène constitué de la séquence GLFEAIAEFIEGGWEGLIEGSAKKK.
